# FASCICULE DE BREVET EUROPEEN

(11) **EP 2 380 882 B1**
(45) Date de publication et mention de la délivrance du brevet: **15.08.2012**
(21) Numéro de dépôt: 09166055.5
(22) Date de dépôt: 30.12.1997
(51) Int. Cl.: C07D 239/60, C07D 285/16, C07D 285/15, C07D 319/06, C07D 327/00, C07D 487/22, C07F 7/18, C07F 17/00, C09K 3/00, C07B 41/00, C08F 4/00, C08J 3/24, C08G 73/02, C08F 220/44, C08G 75/00

(54) **Sels d'anions hétérocycliques, et leurs utilisations comme matériaux à conduction ionique**
Salze von heterocyclischen Anionen und ihre Verwendungen als ionisch leitfähige Materialien
Salts of heterocyclic anions and their uses as ionic conductive materials

(30) Priorité: 30.12.1996 CA 2194127; 05.03.1997 CA 2199231
(43) Date de publication de la demande: 26.10.2011
(62) Demande divisionnaire de: 97403190.8
(73) Titulaire: HYDRO QUEBEC, Montréal QC H2X 3P3 (CA); Centre National pour la Recherche Scientifique (CNRS), 75016 Paris (FR)
(72) Inventeur: Armand, Michel, 75014 Paris (FR); Choquette, Yves, Sainte-Julie Québec J3E 1P4 (CA); Gauthier, Michel, La Prairie Québec J5R 1E6 (CA); Michot, Christophe, Montreal Québec H2T 2Z9 (CA)
(74) Mandataire: Goulard, Sophie

(56) Documents cités:
- FR-A1- 2 606 217
- US-A- 4 105 525
- D.V. PESTOV ET AL.: "Features of the hydrolysis of 5-(anilinomethylene) 1,3-dimethylbarbituric acid", JOURNAL OF GENERAL CHEMISTRY OF THE USSR, vol. 58, no. 8(2), août 1989 (1989-08), pages 1726-7, XP002060789, NEW-YORK, US
- W. KANTLEHNER ET AL.: "Beiträge zur Chemie von Bis(dilakylamino)malonitrilen", LIEBIGS ANNALEN DER CHEMIE, no. 10, octobre 1990 (1990-10), pages 965-73, XP002060791, WEINHEIM, DE
- ILARIA CANDIANI ET AL: "Synthesis of 5-Alkyl substituted Uracil Derivatives from Barbituric Acid", HETEROCYCLES, ELSEVIER SCIENCE PUBLISHERS B.V. AMSTERDAM, NL, vol. 34, no. 5, 1 janvier 1992 (1992-01-01), pages 875-879, XP009150059, ISSN: 0385-5414

## Description

La présente invention a pour objet des composés ioniques dans lesquels la charge anionique est délocalisée, et leurs utilisations.

Les dérivés des anions non nucléophiles ou peu basiques présentent une importance croissante dans toutes les applications de la chimie pour stabiliser ou activer des charges cationiques diverses telles que celles des colorants, des espèces intermédiaires dans les polymérisations, ou celles qui interviennent comme intermédiaires pour des réactions variées de la chimie organique. En électrochimie, il est de plus en plus fait appel à des milieux autres que l'eau pour des applications telles que les générateurs primaires ou secondaires, les supercapacités, les systèmes de modulation de la lumière. L'introduction d'une faible conductivité ionique dans les matériaux usuels (polymères, liquides combustibles), permet de dissiper les charges électrostatiques.

On connaît principalement les dérivés des anions de coordination, de type BF₄⁻, PF₆⁻, AsF₆⁻, mais ceux-ci présentent un stabilité limitée du fait de l'équilibre de dissociation libérant l'anion fluorure et l'acide de Lewis correspondant, les deux amenant des réactions parasites et présentant une toxicité non négligeable. L'anion perchlorate ClO₄⁻ est thermiquement instable et dangereux. On connaît par ailleurs les anions dérivés des perfluoroalkylsulfonates et plus particulièrement des bis (perfluoroalkylsulfonyl)imides qui présentent des propriétés intéressantes. Mais ce type de chimie est relativement difficile à maîtriser, en particulier lors de la préparation des précurseurs de type R_{F}SO₂-.

On connaît par ailleurs la pyrimidinetrione (acide barbiturique) et ses dérivés obtenus en remplaçant un atome d'oxygène par un atome de soufre (acide thiobarbiturique). On connaît aussi la possibilité de former des sels avec la 2,2-diméthyl-1,3-dioxane-4,6-dione ("acide de Meldrum"). Dans les deux cas, les acides sont relativement faibles (pK_{A} de l'ordre de 5 dans l'eau, de l'ordre de 10 dans le dimethylsulfoxyde). Leurs sels ne sont ni facilement solubles ni facilement dissociables dans les solvants organiques. Dans le cas de la pyrimidinetrione, les liaisons hydrogène formées par les protons liés à l'azote renforcent cette insolubilité. Leur substitution par des radicaux alkyles diminue fortement la force de l'acide.

Les inventeurs ont maintenant trouvé que, de manière surprenante, la solubilité et la dissociation des sels obtenus à partir des composés dérivés de la pyrimidinetrione et de ses homologues par des substitutions sur le carbone en position 5, ou sur les azotes en position 1 et 3 étaient considérablement augmentées lorsque les substituants ont un pouvoir attracteur électronique. Il en est de même pour les composés dérivés des 1,3-dioxane-4,6-diones et de leurs homologues qui portent un substituant électroattracteur sur le carbone 2 et/ou sur le carbone 5. Le choix des substituants et les nombreuses combinaisons possibles sur trois sites de substitution pour chaque famille donnent des matériaux variés dont il est possible moduler les propriétés physiques ou chimiques dans une large mesure. Ces composés ont des propriétés intéressantes pour les applications précitées et leur préparation fait appel à des matériaux plus faciles d'accès. Il est notamment possible d'obtenir des hétérocycles anioniques stables incorporant des quantités plus faibles de fluor, ou d'utiliser comme produits de départ des composés fluorés faciles d'accès. Certains composés peuvent éviter totalement le recours à des atomes de fluor.

Un composé de la présente invention comprend au moins une partie anionique associée à au moins une partie cationique M en nombre suffisant pour assurer la neutralité électronique de l'ensemble. Il est caractérisé en ce que M est un hydroxonium, un nitrosonium NO⁺, un ammonium -NH₄⁺, un cation métallique ayant la valence m, un cation organique ayant la valence m ou un cation organométallique ayant la valence m, et en ce que la partie anionique est un hétérocycle aromatique répondant à la formule 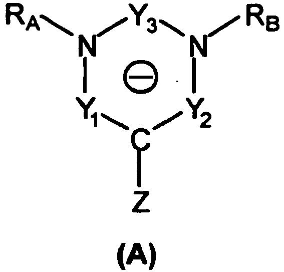 dans laquelle :
- Y₁ et Y₂ représentent chacun un groupement carbonyle,
- Y₃ représente un groupement carbonyle ou un groupement sulfonyle,;
- Z représente un radical électro-attracteur choisi parmi les radicaux R_{E}SO₂ dans lesquels R_{E} représente radical alkyle, alkényle, oxaalkyle, oxaalkényle, azaalkyle, azaalkényle, thiaalkyle, thiaalkényle, aryle, alkylaryle, alkénylaryle, arylalkyle, arylalkényle, un radical alicyclique ou un radical aromatique portant éventuellement au moins une chaîne latérale comprenant un hétéroatome ou comprenant éventuellement au moins un hétéroatome dans le cycle, ledit R_{E} pouvant être halogéné ou perhalogéné ;
- R_{A} et R_{B} représentent indépendamment l'un de l'autre,
   a) un alkyle, un alkényle, un oxa-alkyle, un oxaalkényle, un aza-alkyle, un aza-alkényle, un thia-alkyle, un thia-alkényle, lesdits radicaux portant éventuellement au moins un groupe aryle ;
   b) un aryle portant au moins éventuellement au moins un radical tel que défini en a) ;
   c) un radical alicyclique ou un radical aromatique portant éventuellement au moins une chaîne latérale comprenant un hétéroatome ou comprenant éventuellement au moins un hétéroatome dans le cycle ;
   d) un radical tel que défini ci-dessus en a), b) et c) et portant en outre des atomes d'halogène, sous la forme halogénée ou perhalogénée.

Dans un composé de la présente invention, le cation peut être un cation métallique choisi parmi les cations de métaux alcalins, les cations de métaux alcalino-terreux, les cations de métaux de transition, les cations de métaux trivalents, les cations de terres rares. A titre d'exemple, on peut citer Na⁺, Li⁺, K⁺, Sm³⁺, La³⁺, Ho³⁺ , Sc³⁺ , Al³⁺, Y³⁺, Yb³⁺, Lu³⁺, Eu³⁺.

Le cation peut également être un cation organométallique, notamment un métallocénium. A titre d'exemple, on peut citer les cations dérivés du ferrocène, du titanocène, du zirconocène, d'un indénocénium ou d'un arène métallocénium, les cations des métaux de transition complexés par des ligands de type phosphine possédant éventuellement une chiralité, les cations organométalliques possédant un ou plusieurs groupements alkyles ou aryles fixés d'une manière covalente à un atome ou un groupe d'atome, tels les cations méthylzinc, phénylmercure, trialkylétain ou trialkylplomb. Le cation organo-métallique peut faire partie d'une chaîne polymère.

Selon une variante de l'invention, les composés de l'invention ont un cation organique choisi dans le groupe constitué par les cations R₃O⁺ (oxonium), NR₄⁺ (ammonium), RC (NHR₂) ₂⁺ (amidinium), C (NHR₂)₃⁺ (guanidinium), C₅R₆N⁺ (pyridinium), C₃R₅N₂⁺ (imidazolium), C₃R₇N₂⁺ (imidazolinium), C₂R₄N₃⁺ (triazolium), SR₃⁺ (sulfonium), PR₄⁺ (phosphonium), IR₂⁺ (iodonium), (C₆R₅)₃C⁺ (carbonium). Dans un cation donné, les radicaux R peuvent être tous identiques. Mais un cation peut aussi comporter des radicaux R différents les uns des autres. Un radical R peut être un H ou bien il est choisi parmi les radicaux suivants :
- les radicaux alkyles, alkényles, oxa-alkyles, oxa-alkényles, aza-alkyles, aza-alkényles, thia-alkyles, thia-alkényles, sila-alkyles, sila-alkényles, aryles, arylalkyles, alkyl-aryles, alkényl-aryles, dialkylamino et dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore.

Lorsqu'un cation onium porte au moins deux radicaux R différents de H, ces radicaux peuvent former ensemble un cycle aromatique ou non, englobant éventuellement le centre portant la charge cationique.

Lorsque la partie cationique d'un composé de l'invention est un cation onium, il peut se présenter soit sous la forme d'un groupe cationique indépendant qui n'est lié à la partie anionique que par la liaison ionique entre la charge positive du cation et la charge négative de la partie anionique. Dans ce cas, la partie cationique peut faire partie d'une unité récurrente d'un polymère.

Un cation onium peut également faire partie du radical Z porté par le noyau aromatique anionique. Dans ce cas, un composé de l'invention constitue un zwitterion.

Lorsque le cation d'un composé de l'invention est un cation onium, il peut être choisi de telle sorte à introduire dans le composé des substituants permettant de conférer audit composé des propriétés spécifiques. Par exemple, le cation M⁺ peut être un hétérocycle cationique à caractère aromatique, comportant au moins un atome d'azote alkylé dans le cycle. A titre d'exemple, on peut citer un imidazolium, un triazolium, un pyridinium, un 4-diméthylamino-pyridinium, lesdits cations portant éventuellement un substituant sur les atomes de carbone du cycle. Parmi ces cations, ceux qui donnent un composé ionique selon l'invention dont le point de fusion est inférieur à 150°C sont particulièrement préférés. Un tel composé possédant une température de fusion basse est particulièrement utile pour l'élaboration de matériaux à conduction protonique. Un matériau à conduction protonique particulièrement préféré comprend un composé selon l'invention dans lequel le cation est formé par addition d'un proton sur l'azote d'un imidazole ou d'un triazole, ainsi que la base azotée correspondante dans une proportion de 0,5 à 10 en rapport molaire.

Un composé de l'invention dans lequel le cation M est un groupement cationique possédant une liaison -N=N-, -N=N⁺, un groupement sulfonium, un groupement iodonium, ou un cation arène-ferrocénium substitué ou non, éventuellement incorporé dans une trame polymérique, est intéressant dans la mesure où il est activable par une source d'énergie actinique de longueur d'onde appropriée. Comme exemples particuliers de tels composés, on peut citer ceux dans lesquels le cation est un cation diaryliodonium, un cation dialkylaryliodonium, un cation triarylsulfonium, un cation trialkylaryle sulfonium, ou un cation phénacyl-dialkyl sulfonium substitué ou non. Les cations précités peuvent faire partie d'une chaîne polymère.

Le cation M d'un composé de l'invention peut incorporer un groupement 2,2'[Azobis(2-2'-imidazolinio-2-yl)propane]²⁺ ou 2,2'-Azobis (2-amidiniopropane)²⁺. Le composé de l'invention est alors capable de libérer, sous l'action de la chaleur ou d'un rayonnement ionisant, des radicaux qui permettent d'initier des réactions de polymérisation, de réticulation ou, d'une manière générale, des réactions chimiques mettant en jeu des radicaux libres. De plus, ces composés sont aisément solubles dans les solvants organiques polymères et monomères même de faible polarité, contrairement aux dérivés des anions de type Cl⁻ habituellement associés à ce type de composés. Ils présentent par ailleurs une pression de vapeur négligeable contrairement aux autres amorceurs radicalaires de type peroxyde ou azo, ce qui est un avantage considérable pour la mise en oeuvre de polymères en films minces, la volatilité de l'amorceur ayant pour conséquence une mauvaise polymérisation ou réticulation de la surface du film.

Le choix des substituants permet d'ajuster les propriétés d'un composé ionique de l'invention.

Parmi les radicaux R_{A} et R_{B}, on préfère tout particulièrement les radicaux alkyles et les radicaux alkényles ayant de 1 à 10 atomes de carbone, les radicaux alkyles ou alkényles halogénés ou perhalogénés ayant de 1 à 10 atomes de carbone, et les radicaux oxa-alkyles ou oxa-alkényles ayant de 1 à 10 atomes de carbone.

Un substituant R_{E} peut représenter un radical alkyle, alkényle, oxaalkyle, oxaalkényle, azaalkyle, azaalkényle, thiaalkyle, thiaalkényle, aryle, alkylaryle, alkénylaryle, arylalkyle, arylalkényle, un radical alicyclique ou un radical aromatique portant éventuellement au moins une chaîne latérale comprenant un hétéroatome ou comprenant éventuellement au moins un hétéroatome dans le cycle, ledit R_{E} pouvant être halogéné ou perhalogéné.

Dans un autre mode de réalisation, R_{E} est choisi parmi les radicaux alkyles ou alkényles ayant de 1 à 12 atomes de carbone et comprenant éventuellement au moins un hétéroatome O, N ou S dans la chaîne principale ou dans une chaîne latérale, et portant un groupe hydroxy, un groupe carbonyle, un groupe amine, un groupe carboxyle.

R_{E} peut également être choisi indépendamment l'un de l'autre parmi les radicaux aryle, arylalkyle, alkylaryle ou alkénylaryle, dans lesquels les noyaux aromatiques, éventuellement condensés, comprennent des hétéroatomes tels que l'azote, l'oxygène, le soufre.

Dans un mode de réalisation particulier, R_{E} peut être un radical possédant un groupement iodonium, un groupement sulfonium, oxonium, ammonium, amidinium, guanidinium, pyridinium, imidazolium, imidazolinium, triazolium, phosphonium ou carbonium, ledit groupement ionique jouant totalement ou partiellement le rôle du cation M. Le composé de l'invention constitue alors un zwitterion.

Lorsque R_{E} comporte au moins une insaturation éthylénique et/ou un groupe condensable et/ou un groupe dissociable par voie thermique, par voie photochimique ou par dissociation ionique, les composés de l'invention sont des composés réactifs qui peuvent être soumis à des polymérisations, des réticulations ou des condensations, éventuellement avec d'autres monomères. Ils peuvent également être utilisés pour fixer des groupes ionophores sur les polymères portant la fonction réactive.

Un substituant R_{E} peut être un groupe mésomorphe ou un groupe chromophore ou un polymère conducteur électronique autodopé ou un alcoxysilane hydrolysable.

Un substituant R_{E} peut comporter un groupement susceptible de piéger les radicaux libres tel que par exemple un phénol encombré ou une quinone.

Un substituant R_{E} peut aussi comporter un dipôle dissociant tel que, par exemple, une fonction amide, une fonction sulfonamide ou une fonction nitrile.

Un substituant R_{E} peut aussi comporter un couple rédox, par exemple un groupe disulfure, un groupe thioamide, un groupe ferrocène, un groupe phénothiazine, un groupe bis(dialkylaminoaryle), un groupe nitroxyde ou un groupe imide aromatique.

Un substituant R_{E} peut comporter ou un ligand complexant ou un groupe optiquement actif.

Selon une autre variante, un composé selon l'invention comprend un substituant Z qui représente un radical ayant une valence v supérieure à deux, comportant lui-même au moins l'un des groupements hétérocycliques aromatiques anioniques

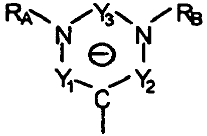

Dans ce cas, les charges négatives présentes sur la partie anionique du composé de l'invention devront être compensées par le nombre approprié de cations ou de groupes ionophores cationiques M.

Dans un mode de réalisation particulier, le radical Z multivalent est un radical bivalent comprenant au moins un groupe -SO₂-, un groupe -CO-, un groupe perfluoroalkylène ayant de 2 à 8 atomes de carbone, un groupe phénylène éventuellement substitué par des hétéroatomes, un groupement rédox -(W=W)ₙ ou un groupement cationique -(W=W)ₙ-W⁺-, dans lesquels W représente un atome d'azote ou un groupement -C(R)-, R représentant un atome d'hydrogène ou un radical organique et 0≤n≤5. La présence du groupement cationique -(W=W)ₙ-W⁺- confère au composé de l'invention des propriétés de colorant très utiles pour les lasers. R a de préférence de 1 à 8 atomes de carbone, ou bien deux radicaux R portés par des atomes de carbone adjacents formant un cycle. Dans un autre mode de réalisation, Z est partie d'une unité récurrente d'une chaîne polymère. Le composé de l'invention présente alors des propriétés de polyélectrolyte.

R_{E} peut également faire partie d'un radical poly(oxyalkylène) ou d'un radical polystyrène.

Un composé de la présente invention dans lequel l'anion répond à la formule générale A ci-dessus peut être préparé selon les schémas réactionnels suivants : 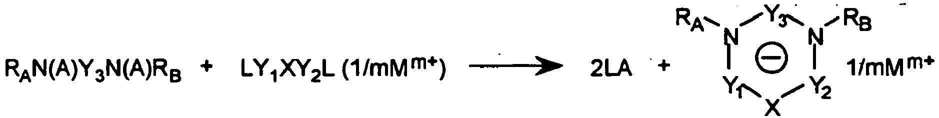 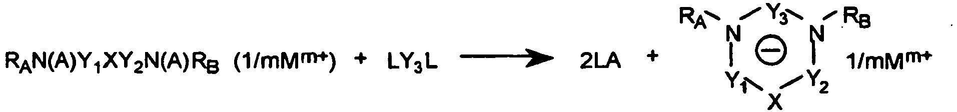 Dans tous les cas :
- L: représente un groupe partant électronégatif choisi parmi F, Cl, Br, N-imidazoyle, N-triazoyle, R_{F}-O-, R_{F}CH₂-O- et R_{F}SOₓ-, R_{F} étant un radical perfluoralkyle ;
- A: représente un cation M⁺, un groupe trialkylsilyle, un groupe trialkyle germanyle, un groupe trialkylstannyle ou un groupe tertioalkyle, dans lesquels les substituants alkyles ont de 1 à 6 atomes de carbone.

Il est avantageux dans le cas ou A = H de permettre le déplacement de la réaction dans le sens de la formation du composé de l'invention par addition d'une base tertiaire ou encombrée T susceptible de former le sel L⁻[HT⁺] par combinaison avec le proton.

Les bases tertiaires préférées sont en particulier choisies parmi les alkylamines, par exemple la triéthylamine, la di-isopropyléthylamine, la quinuclidine ; le 1,4 diazabicyclo[2,2,2]octane (DABCO) ; les pyridines, par exemple la pyridine, les alkylpyridines, les dialkylaminopyridines ; les imidazoles, par exemple les N-alkylimidazoles, l'imidazo[1,2-a]pyridine ; les amidines, par exemple le 1,5 diazabicyclo-[4,3,0]non-5-ène (DBN), le 1,8 diazabicyclo[5, 4, 0]undec-7-ène (DBU) ; les guanidines, par exemple la tétraméthyl guanidine, la 1,3,4,7,8-hexahydro-1-méthyl-2H-pyrimido[1,2-a]pyrimidine (HPP).

Dans tous ces composés, X représente -CH- ou -C(Z)-. Les composés obtenus avec X = CH peuvent ensuite être modifiés par subsitution du proton résiduel, par exemple par action de l'anhydride trifluorométhane sulfonique.

Dans les cas où au moins un des Yᵢ est un groupement -C(=NCN)- ou un groupement -C(=C(CN)₂)-, les procédés pour obtenir ces groupements sont connus de l'homme de métier. A titre d'exemple, on peut citer la réaction d'un groupement carbonyle avec la cyanamide ou le malononitrile.

Les composés ioniques de la présente invention comprennent au moins un groupement ionophore sur lequel sont fixés des substituants qui peuvent être très variés. Compte tenu du grand choix possible pour les substituants, les composés de l'invention permettent d'induire des propriétés de conduction ionique dans la plupart des milieux organiques, liquides ou polymères possédant une polarité, même faible. Les applications sont importantes dans le domaine de l'électrochimie, en particulier du stockage de l'énergie dans des générateurs primaires ou secondaires, dans les supercapacités, dans les piles à combustibles et dans les diodes électroluminescentes. La compatibilité des composés ioniques de l'invention avec les polymères ou les liquides organiques permet d'induire des propriétés antistatiques marquées, même lorsque la teneur en composé ionique est extrêmement faible. Les composés de l'invention qui sont des polymères, de même que des composés polymères obtenus à partir de composés de l'invention ayant la propriété de se polymériser ou de se copolymériser, présentent les propriétés énumérées ci-dessus avec l'avantage d'avoir d'une charge anionique immobile. C'est pourquoi un autre objet de la présente invention est constitué par un matériau à conduction ionique constitué par un composé ionique de la présente invention en solution dans un solvant.

Dans un mode de réalisation, le composé ionique utilisé pour l'élaboration d'un matériau à conduction ionique est choisi parmi les composés dont le cation est l'ammonium, ou un cation dérivé d'un métal, en particulier le lithium ou le potassium, le zinc, le calcium, les métaux des terres rares, ou un cation organique, tel qu'un ammonium substitué, un imidazolium, un triazolium, un pyridinium, un 4-diméthylamino-pyridinium, lesdits cations portant éventuellement un substituant sur les atomes de carbone du cycle. Le matériau à conduction ionique ainsi obtenu présente une conductivité et une solubilité dans les solvants élevées, du fait des interactions faibles entre la charge positive et la charge négative. Son domaine de stabilité électrochimique est étendu, et il est stable dans des milieux aussi bien réducteurs qu'oxydants. De plus, les composés qui ont un cation organique et un point de fusion inférieur à 150°C, en particulier les composés de imidazolium, de triazolium, de pyridinium, de 4-diméthylamino-pyridinium présentent une conductivité élevée intrinsèque, même en l'absence de solvant, lorsqu'ils sont en phase fondue.

Les propriétés du matériau à conduction ionique peuvent également être adaptées par le choix du substituant R_{A} et R_{B}.

Le choix pour l'un au moins des substituants R_{A} ou R_{B} d'un groupe alkyle, d'un groupe aryle, d'un groupe alkylaryle ou d'un groupe arylalkyle, permet d'induire dans le matériau à conduction ionique des propriétés de type mésogène, en particulier les groupements alkyles de 6 à 20 atomes de carbones, les groupements aryle-alkyle, en particulier ceux contenant l'entité biphényle qui forment des phases de type cristal liquide. Des propriétés de conduction dans des phases de type cristal liquide, nématique, cholestérique ou discotique, sont intéressantes pour les applications relatives à l'affichage optique ou pour réduire la mobilité des anions dans les électrolytes, en particulier dans les électrolytes polymères, sans affecter la mobilité des cations. Cette particularité est importante pour les applications dans les générateurs électrochimiques, en particulier ceux mettant en jeu les cations lithium.

Lorsque le substituant Z est un groupe mésomorphe ou un groupe comprenant au moins une insaturation éthylénique et/ou un groupe condensable et/ou un groupe dissociable par voie thermique, par voie photochimique ou par dissociation ionique, le matériau à conduction ionique forme aisément des polymères ou copolymères qui sont des polyélectrolytes, soit intrinsèques quand le polymère porte des groupements solvatants, soit par addition d'un solvant polaire de type liquide ou polymère, ou par mélange avec un tel solvant. Ces produits ont une conductivité uniquement due au cations, ce qui constitue une propriété très utile dans les applications de type générateur électrochmique. En faible fraction molaire dans un copolymère, ils induisent des propriétés antistatiques stables et peu dépendantes de l'humidité et favorisent la fixation de colorants cationiques, cette propriété étant utile pour les fibres textiles et les lasers à colorants.

La présence d'un substituant Z qui est un polymère conducteur électronique autodopé, améliore la stabilité du matériau à conduction ionique par rapport aux agents extérieurs. La conductivité est stable dans le temps même à des températures élévées. En contact avec les métaux, ces matériaux donnent des résistances d'interface très faibles et protègent en particulier les métaux ferreux ou l'aluminium de la corrosion.

Lorsque Z est un alcoxysilane hydrolysable, le matériau à conduction ionique peut former des des polymères stables par simple mécanisme d'hydrolyse-condensation en présence d'eau, permettant ainsi de traiter les surfaces d'oxydes, de silice, de silicates, en particulier le verre, pour induire des propriétés de conduction de surface, des propriétés antistatiques, ou pour favoriser l'adhésion de polymères polaires.

Lorsque Z est un groupe comprenant un piège à radicaux libres tel qu'un phénol encombré, ou une quinone, le matériau à conduction ionique présente les avantages et propriétés suivantes : il agit comme antioxydant ne présentant pas de volatilité et compatible avec les monomères et polymères polaires, auquel il confère de surcroît des propriétés antistatiques.

Lorsque Z comprend un dipôle dissociant tel qu'une amide, une sulfonamide ou un nitrile, le matériau à conduction ionique a une conductivité améliorée dans des milieux de faible et moyenne polarité, en particulier dans les polymères solvatants, ce qui permet de minimiser, voire de supprimer l'addition de solvants ou de plastifiants volatils.

La présence d'un substituant Z qui contient un couple rédox tel qu'un disulfure, une thioamide, un ferrocène, une phéno-thiazine, un groupe bis(dialkylaminoaryle), un nitroxyde, un imide aromatique, permet d'induire dans le matériau à conduction ionique des propriétés de navette rédox utiles comme élément de protection et d'égalisation de charge des générateurs électrochimiques, dans les systèmes photoélectrochimiques, en particulier de conversion de la lumière en électricité, dans les systèmes de modulation de la lumière de type électrochrome.

La présence d'un substituant Z qui est un ligand complexant dans un matériau à conduction ionique permet de chélater les cations métalliques, en particulier ceux qui possèdent un charge élevée (2, 3 et 4), sous forme de complexe soluble dans les milieux organiques, y compris dans les milieux aprotiques, et permet le transport de ces cations en particulier sous forme de complexe anionique, dans les polymères solvatants. Les cations métalliques de charge élevée sont en effet immobiles dans les polymères solvatants. Ce type de complexant donne avec certains cations des métaux de transition (Fe, Co...) ou de certaines terres rares (Ce, Eu...) des couples rédox particulièrement stables.

Les matériaux à conduction ionique contenant un composé de l'invention dans l'un au moins des substituants R_{A} ou R_{B} est un substituant alkyle ou alkényle qui contient au moins un hétéroatome choisi parmi O, N et S ont un pouvoir complexant, et plastifiant, en particulier dans les polymères polaires et tout spécialement les polyéthers. Les hétéroatomes N et S sont sélectivement complexants pour les cations des métaux de transition, Zn et Pb.

Lorsque le matériau à conduction ionique contient un composé de l'invention dans lequel Z représente une unité récurrente d'une chaîne polymère, le matériau constitue un polyélectrolyte.

Un matériau à conduction ionique de la présente invention comprend un composé ionique de l'invention en solution dans un solvant.

Le solvant peut être un solvant liquide aprotique, un polymère polaire ou un de leurs mélanges.

Le solvant liquide aprotique est choisi par exemple parmi les éthers linéaires et les éthers cycliques, les esters, les nitriles, les dérivés nitrés, les amides, les sulfones, les sulfolanes, les alkylsulfamides et les hydrocarbures partiellement halogénés. Les solvants particulièrement préférés sont le diéthyléther, le diméthoxyéthane, le glyme, le tétrahydrofurane, le dioxane, le diméthyltétrahydrofurane, le formiate de méthyle ou d'éthyle, le carbonate de propylène ou d'éthylène, les carbonates d'alkyles (notamment le carbonate de diméthyle, le carbonate de diéthyle et le carbonate de méthylpropyle), les butyrolactones, l'acétonitrile, le benzonitrile, le nitrométhane, le nitrobenzène, la diméthylformamide, la diéthylformamide, la N-méthyl-pyrrolidone, la diméthylsulfone, la tétraméthylène sulfone et les tétraalkylsulfonamides ayant de 5 à 10 atomes de carbone.

Le polymère polaire peut être choisi parmi les polymères solvatants, réticulés ou non, portant ou non des groupes ioniques greffés. Un polymère solvatant est un polymère qui comporte des unités solvatantes contenant au moins un hétéroatome choisi parmi le soufre, l'oxygène, l'azote et le fluor. A titre d'exemple de polymères solvatants, on peut citer les polyéthers de structure linéaire, peigne ou à blocs, formant ou non un réseau, à base de poly(oxyde d'éthylène), ou les copolymères contenant le motif oxyde d'éthylène ou oxyde de propylène ou allylglycidyléther, les polyphosphazènes, les réseaux réticulés à base de polyéthylène glycol réticulé par des isocyanates ou les réseaux obtenus par polycondensation et portant des groupements qui permettent l'incorporation de groupements réticulables. On peut également citer les copolymères à blocs dans lesquels certains blocs portent des fonctions qui ont des propriétés rédox. Bien entendu, la liste ci-dessus n'est pas limitative, et tous les polymères présentant des propriétés solvatantes peuvent être utilisés.

Un matériau à conduction ionique de la présente invention peut comprendre simultanément un solvant liquide aprotique choisi parmi les solvants liquides aprotiques cités ci-dessus et un solvant polymère polaire comprenant des unités contenant au moins un hétéroatome choisi parmi le soufre, l'azote, l'oxygène et le fluor. Il peut comprendre de 2 à 98% de solvant liquide. A titre d'exemple d'un tel polymère polaire, on peut citer les polymères qui contiennent principalement des unités dérivées de l'acrylonitrile, du fluorure de vinylidène, de la N-vinylpyrrolidone ou du méthacrylate de méthyle. La proportion de liquide aprotique dans le solvant peut varier de 2% (correspondant à un solvant plastifié) à 98% (correspondant à un solvant gélifié).

Un matériau à conduction ionique de la présente invention peut contenir en outre un sel utilisé classiquement dans l'art antérieur pour l'élaboration d'un matériau à conduction ionique. Parmi les sels utilisables en mélange avec un composé ionique selon l'invention, on préfère tout particulièrement un sel choisi parmi les perfluoroalcanesulfonates, les bis(perfluoroalkylsulfonyl) imidures, les bis(perfluoroalkyl-sulfonyl) méthanes et les tris(perfluoroalkylsulfonyl)-méthanes.

Bien entendu, un matériau à conduction ionique de l'invention peut contenir en outre les additifs utilisés de manière classique dans ce type de matériau, et notamment des charges minérales ou organiques sous forme de poudre ou de fibres.

Un matériau à conduction ionique de l'invention peut être utilisé comme électrolyte dans un générateur électrochimique. La présente invention a ainsi pour autre objet un générateur électrochimique comprenant une électrode négative et une électrode positive séparées par un électrolyte, caractérisé en ce que l'électrolyte est un matériau à conduction ionique tel que défini ci-dessus. Selon un mode de réalisation particulier, un tel générateur comprend une électrode négative constituée par du lithium métallique, ou par l'un de ses alliages, éventuellement sous forme de dispersion nanométrique dans de l'oxyde de lithium, ou par un nitrure double de lithium et d'un métal de transition, ou par un oxyde à bas potentiel ayant pour formule générale Li_{1+y+x/3}Ti_{2-x/3}O₄ (0 ≤ x ≤ 1, 0 ≤ y ≤ 1), ou par le carbone et les produit carbonés issus de la pyrolyse de matières organiques. Selon un autre mode de réalisation, le générateur comprend une électrode positive choisie parmi les oxydes de vanadium VOₓ (2 ≤ x ≤ 2, 5), LiV₃O₈, Li_{y}Ni₁₋ₓCoₓO₂, (0 ≤ x ≤ 1 ; 0 ≤ y ≤ 1), les spinelles de manganèse Li_{y}Mn₁₋ₓMₓO₂ (M = Cr, Al, V, Ni, 0 ≤ x ≤ 0,5 ; 0 ≤ y ≤ 2), les polydisulfures organiques, FeS, FeS₂, le sulfate de fer Fe₂(SO₄)₃, les phosphates et phosphosilicates de fer et de lithium de structure olivine, ou leurs produits de substitution du fer par le manganèse, utilisés seuls ou en mélanges. Le collecteur de l'électrode positive est de préférence en aluminium.

Un matériau à conduction ionique de la présente invention peut également être utilisé dans une supercapacité. Un autre objet de la présente invention est par conséquent une supercapacité utilisant au moins une électrode de carbone à haute surface spécifique, ou une électrode contenant un polymère rédox, dans laquelle l'électrolyte est un matériau à conduction ionique tel que défini ci-dessus.

Un matériau à conduction ionique de la présente invention peut également être utilisé pour le dopage p ou n d'un polymère à conduction électronique et cette utilisation constitue un autre objet de la présente invention.

En outre, un matériau à conduction ionique de la présente invention peut être utilisé comme électrolyte dans un dispositif électrochrome. Un dispositif électrochrome dans lequel l'électrolyte est un matériau à conduction ionique selon l'invention est un autre objet de la présente invention.

Il a été observé que la forte dissociation des espèces ioniques des composés de l'invention se traduisait par une stabilisation des carbocations, en particulier ceux dans lesquels il existe une conjugaison avec l'oxygène ou l'azote et, d'une manière surprenante, par une forte activité de la forme protonée des composés l'invention sur certains monomères. La présente invention a donc également pour objet l'utilisation des composés ioniques comme photoinitiateurs sources d'acides de Brønsted catalyseurs de polymérisation ou de réticulation de monomères ou de prépolymères capables de réagir par voie cationique, ou comme catalyseurs pour la modification de polymères.

Le procédé de polymérisation ou de réticulation de monomères ou de prépolymères capables de réagir par voie cationique est caractérisé en ce que l'on utilise un composé de l'invention comme photoinitiateur source d'acide catalysant la réaction de polymérisation. Les composés selon l'invention dans lesquels le cation est un groupement possédant une liaison -N=N⁺, -N=N- un groupement sulfonium, un groupement iodonium, ou un cation arène-ferrocénium substitué ou non, éventuellement incorporé dans une trame polymérique, sont particulièrement préférés.

Le choix des divers substituants de l'hétérocycle anionique est effectué de manière à augmenter la solubilité dudit composé dans les solvants utilisés pour la réaction des monomères ou des prépolymères, et en fonction des propriétés souhaitées pour le polymère final. Par exemple, le choix de radicaux alkyles non substitués donne une solubilité dans les milieux peu polaires. Le choix de radicaux comprenant un groupe oxa ou une sulfone donnera une solubilité dans les milieux polaires. Les radicaux incluant un groupement sulfoxyde, un groupement sulfone, un groupement oxyde de phosphine, un groupement phosphonate, obtenus respectivement par addition d'oxygène sur les atomes de soufre ou de phosphore, peuvent conférer au polymère obtenu des propriétés améliorées en ce qui concerne l'adhésion, la brillance, la résistance à l'oxydation ou aux UV. Les monomères et les prépolymères qui peuvent être polymérisés ou réticulés à l'aide des photoinitiateurs de la présente invention sont ceux qui peuvent subir une polymérisation cationique.

Parmi les monomères, on peut citer les monomères qui comportent une fonction éther cyclique, une fonction thioéther cyclique ou une fonction amine cyclique les composés vinyliques (plus particulièrement les éthers vinyliques), les oxazolines, les lactones et les lactames.

Parmi les monomères du type éther ou thioéther cyclique, on peut citer l'oxyde d'éthylène, l'oxyde de propylène, l'oxétane, l'épichlorhydrine, le tétrahydrofurane, l'oxyde de styrène, l'oxyde de cyclohexène, l'oxyde de vinylcyclohexène, le glycidol, l'oxyde de butylène, l'oxyde d'octylène, les éthers et les esters de glycidyle (par exemple le méthacrylate ou l'acrylate de glycidyle, le phényl glycidyl éther, le diglycidyléther de bisphénol A ou ses dérivés fluorés), les acétals cycliques ayant de 4 à 15 atomes de carbone (par exemple le dioxolane, le 1,3-dioxane, le 1,3-dioxépane) et les spiro-bicyclo dioxolanes.

Parmi les composés vinyliques, les éthers vinyliques constituent une famille très importante de monomères sensibles en polymérisation cationique. A titre d'exemple, on peut citer l'éthyl vinyl éther, le propyl vinyl éther, l'isobutyl vinyl éther, l'octadécyl vinyl éther, l'éthylèneglycol monovinyl éther, le diéthylèneglycol divinyl éther, le butanediol monovinyl éther, le butanediol divinyl éther, l'hexanediol divinyl éther, l'éthylèneglycol butyl vinyl éther, le triéthylèneglycol méthyl vinyl éther, le cyclohexanediméthano monovinyl éther, le cyclohexanediméthanol divinyl éther, le 2-éthylhexyl vinyl éther, le poly-THF-divinyl éther ayant une masse comprise entre 150 et 5000, le diéthylèneglycol monovinyl éther, le triméthylolpropane trivinyl éther, l'aminopropyl vinyl éther, le 2-diéthylaminoéthyl vinyl éther.

Comme autres composés vinyliques, on peut citer à titre d'exemple les 1,1-dialkyléthylènes (par exemple l'isobutène), les monomères aromatiques vinyliques (par exemple le styrène, les α-alkylstyrènes, notamment l'α-méthylstyrène, le 4-vinylanisole, l'acénaphtène), les composés N-vinyliques (par exemple la N-vinylpyrolidone ou les N-vinyl sulfonamides).

Parmi les prépolymères, on peut citer les composés dans lesquels des groupements époxy sont portés par une chaîne aliphatique, une chaîne aromatique, ou une chaîne hétérocyclique, par exemple les éthers glycidiques du bisphénol A éthoxylés par 3 à 15 unités d'oxyde d'éthylène, les siloxanes possédant des groupements latéraux du type époxycyclohexène-éthyle obtenus par hydrosilylation des copolymères de di alkyl, d'alkylaryle ou de diaryl siloxane avec le méthyl hydrogénosiloxane en présence d'oxyde de vinylcyclohexène, les produits de condensation du type sol-gel obtenus à partir du triéthoxy ou du triméthoxy silapropylcyclohexène oxyde, les uréthanes incorporant les produits de réaction du butanediol monovinyléther et d'un alcool de fonctionnalité supérieure ou égale à 2 sur un di ou un tri isocyanate aliphatique ou aromatique.

Le procédé de polymérisation selon l'invention consiste à mélanger au moins un monomère ou prépolymère capable de polymériser par voie cationique et au moins un composé ionique de l'invention, et à soumettre le mélange obtenu à un rayonnement actinique ou un rayonnement β. De préférence, le mélange réactionnel est soumis au rayonnement après avoir été mis sous forme d'une couche mince ayant une épaisseur inférieure à 5 mm, de préférence sous forme d'un film mince ayant une épaisseur inférieure ou égale à 500 µm. La durée de la réaction dépend de l'épaisseur de l'échantillon et dé la puissance de la source à la longueur d'onde λ active. Elle est définie par la vitesse de défilement devant la source, qui est comprise entre 300 m/min et 1 cm/min. Des couches de matériau final ayant une épaisseur supérieure à 5 mm peuvent être obtenues en répétant plusieurs fois l'opération consistant à épandre une couche et à la traiter par le rayonnement.

Généralement, la quantité de photoinitiateur utilisé est comprise entre 0,01 et 15 % en poids par rapport au poids de monomère ou de prépolymère, dé préférence entre 0,1 et 5 % en poids.

Un composé ionique de la présente invention peut être utilisé comme photoinitiateur en l'absence de solvant, notamment lorsque l'on souhaite polymériser des monomères liquides dans lesquels le composé ionique utilisé comme photoinitiateur est soluble ou aisément dispersable. Cette forme d'utilisation est particulièrement intéressante, car elle permet de supprimer les problèmes liés aux solvants (toxicité, inflammabilité).

Un composé ionique de la présente invention peut également être utilisé en tant que photoinitiateur sous forme d'une solution homogène dans un solvant inerte vis-à-vis de la polymérisation, prête à l'emploi et aisément dispersable, en particulier dans le cas où le milieu à polymériser ou à réticuler présente une viscosité élevée.

Comme exemple de solvant inerte, on peut citer les solvants volatils, tels que l'acétone, la méthyl-éthyl cétone et l'acétonitrile. Ces solvants serviront simplement à diluer les produits à polymériser ou à réticuler (pour les rendre moins visqueux, surtout lorsqu'il s'agit d'un prépolymère). Ils seront éliminés après la polymérisation ou la réticulation par séchage. On peut également citer les solvants non volatils. Un solvant non volatil sert également à diluer les produits que l'on veut polymériser ou réticuler, et à dissoudre le composé ionique de l'invention utilisé comme photoinitiateur, mais il restera dans le matériau formé et il agit ainsi comme plastifiant. A titre d'exemple, on peut citer le carbonate de propylène, la γ-butyrolactone, les éther-esters des mono-, di-, tri- éthylène ou propylène glycols, les éther-alcools des mono-, di-, tri- éthylène ou propylène glycols, les plastifiants tels que les esters de l'acide phtalique ou de l'acide citrique.

Dans un autre mode de mise en oeuvre de l'invention, on utilise comme solvant ou diluant un composé réactif vis-à-vis de la polymérisation, qui est un composé de faible masse moléculaire et de faible viscosité qui va jouer à la fois le rôle de monomère polymérisable et le rôle de solvant ou de diluant pour des monomères plus visqueux ou des prépolymères utilisés conjointement. Après la réaction, ces monomères ayant servi de solvant font partie du réseau macromoléculaire finalement obtenu, leur intégration étant plus grande lorsqu'il s'agit de monomères bi-fonctionnels. Le matériau obtenu après irradiation ne contient plus de produits ayant un faible poids moléculaire et une tension de vapeur appréciable, ou susceptibles de contaminer les objets avec lesquels le polymère est en contact. A titre d'exemple, un solvant réactif peut être choisi parmi les mono et di éthers vinyliques des mono-, di-, tri-, tétra- éthylène et propylène glycols, la N-méthylpyrolidone, le 2-propényléther du carbonate de propylène commercialisé par exemple sous la dénomination PEPC par la société ISP, New Jersey, Etats-Unis.

Pour irradier le mélange réactionnel, le rayonnement peut être choisi parmi le rayonnement ultraviolet, le rayonnement visible, les rayons X, les rayons y et le rayonnement β. Lorsque l'on utilise la lumière ultraviolette comme rayonnement actinique, il peut être avantageux d'ajouter aux photoinitiateurs de l'invention des photosensibilisateurs destinés à permettre une photolyse efficace avec les longueurs d'ondes moins énergétiques que celles correspondant au maximum d'absorption du photoinitiateur, telles que celles émises par les dispositifs industriels, (λ ≈ 300 nm pour les lampes à vapeur de mercure en particulier). De tels additifs sont connus, et à titre d'exemples non limitatifs, on peut citer l'anthracène, le diphényl-9,10-anthracène, le pérylène, la phénothiazine, le tétracène, la xanthone, la thioxanthone, l'acétophénone, la benzophénone, les 1,3,5-triaryl-2-pyrazolines et leurs dérivés, en particulier les dérivés de substitution sur les noyaux aromatiques par des radicaux alkyles, oxa- ou aza-alkyles permettant entre autre de changer la longueur d'onde d'absorption. L'isopropylthioxantone est un exemple de photosensibilisateur préféré lorsque l'on utilise un sel d'iodonium selon l'invention comme photoinitiateur.

Parmi les différents types de rayonnement mentionnés, le rayonnement ultraviolet est particulière préféré. D'une part, il est plus commode d'emploi que les autres rayonnements mentionnés. D'autre part, les photoinitiateurs sont en général directement sensibles aux rayons UV et les photosensibilisateurs sont d'autant plus efficaces que la différence d'énergie (δλ) est plus faible.

Les composés ioniques de l'invention peuvent aussi être mis en oeuvre en association avec des amorceurs de type radicalaire générés thermiquement ou par action d'une radiation actinique. Il est ainsi possible de polymériser ou de réticuler des mélanges de monomères ou de prépolymères contenant des fonctions dont les modes de polymérisation sont différents, par exemple des monomères ou des prépolymères polymérisant par voie radicalaire et des monomères ou des prépolymères polymérisant par voie cationique. Cette possibilité est particulièrement avantageuse pour créer des réseaux interpénétrés ayant des propriétés physiques différentes de celles qui seraient obtenues par simple mélange des polymères issus des monomères correspondants. Les éthers vinyliques ne sont pas ou sont peu actifs par amorçage radicalaire. Il est donc possible, dans un mélange réactionnel contenant un photoinitiateur selon l'invention, un amorceur radicalaire, au moins un monomère du type éther vinylique et au moins un monomère comprenant des doubles liaisons non activées telles que celles des groupes allyliques, d'effectuer une polymérisation séparée de chaque type de monomère. Il est par contre connu que les monomères déficients en électrons, tels que les esters ou les amides de l'acide fumarique, de l'acide maléique, de l'acide acrylique ou méthacrylique, de l'acide itaconique, de l'acrylonitrile, du méthacrylonitrile, la maléimide et ses dérivés, forment en présence d'éthers vinyliques riches en électrons, des complexes de transfert de charge donnant des polymères alternés 1:1 par amorçage radicalaire. Un excès initial de monomères vinyliques par rapport à cette stoechiométrie permet de préserver des fonctions polymérisables par initiation cationique pure. Le déclenchement de l'activité d'un mélange d'amorceur radicalaire et d'amorceur cationique selon l'invention peut être fait simultanément pour les deux réactifs dans le cas par exemple d'insolation par un rayonnement actinique d'une longueur d'onde pour laquelle les photoinitiateurs de l'invention et les amorceurs radicalaires choisis sont actifs, par exemple à λ = 250 nm. A titre d'exemple, on peut citer comme amorceurs les produits commerciaux suivants : Irgacure 184^{®}, Irgacure 651^{®}, Irgacure 261^{®} Quantacure DMB^{®}, Quantacure ITX^{®}.

Il peut aussi être avantageux d'utiliser les deux modes de polymérisation d'une manière séquentielle, pour former dans un premier temps des prépolymères dont la mise en forme est aisée et dont le durcissement, l'adhésion, la solubilité ainsi que le degré de réticulation peuvent être modifiés par le déclenchement de l'activité de l'amorceur cationique. Par exemple, un mélange d'un amorceur radicalaire thermodissociable et d'un photoinitiateur cationique selon l'invention permet de réaliser des polymérisations ou des réticulations séquentielles, d'abord sous l'action de la chaleur, puis sous l'action d'un rayonnement actinique. D'une manière similaire, si l'on choisit un amorceur radicalaire et un photoinitiateur cationique selon l'invention, le premier étant photosensible à des longueurs d'ondes plus longues que celle déclenchant le photoinitiateur selon l'invention, on obtient une réticulation en deux étapes contrôlables. Des amorceurs radicalaires peuvent être par exemple Irgacure® 651 permettant d'amorcer des polymérisations radicalaires à des longueurs d'onde de 365 nm.

L'invention a également pour objet l'utilisation des composés ioniques de l'invention pour les réactions d'amplification chimique de photoresists pour la microlithographie. Lors d'une telle utilisation, un film d'un matériau comprenant un polymère et un composé ionique de l'invention est soumis à une irradiation. L'irradiation provoque la formation de l'acide par remplacement du cation M par un proton, qui catalyse la décomposition ou la transformation du polymère. Après décomposition ou transformation du polymère sur les parties du film qui ont été irradiées, les monomères formés ou le polymère transformé sont éliminés et il reste une image des parties non exposées. Pour cette application particulière, il est avantageux d'utiliser un composé de l'invention qui se présente sous la forme d'un polymère constitué essentiellement d'unités récurrentes styrényles portant un hétérocycle aromatique anionique comme substituant. Ces composés permettent d'obtenir après photolyse des produits qui ne sont pas volatils, et donc pas odorants lorsqu'il s'agit de sulfures. Parmi les polymères qui peuvent ainsi être modifiés en présence d'un composé de l'invention, on peut citer notamment les polymères contenant des motifs ester ou des motifs aryléther de tertioalkyle, par exemple les poly(phtalaldéhydes), les polymères de bisphénol A et d'un diacide, le polytertiobutoxycarbonyl oxystyrène, le polytertiobutoxy-α-méthyl styrène, le polyditertiobutylfumarate-co-allyltriméthylsilane et les polyacrylates d'un alcool tertiaire, en particulier le polyacrylate de tertiobutyle. D'autres polymères sont décrits dans J.V. Crivello et al, Chemistry of Materials 8, 376-381, (1996).

Les composés ioniques de la présente invention, qui présentent une grande stabilité thermique, offrent de nombreux avantages par rapport aux sels connus de l'art antérieur. Ils ont notamment des vitesses d'amorçage et de propagation comparables ou supérieures à celles obtenues à l'aide des anions de coordination de type PF₆⁻, AsF₆⁻ et surtout SbF₆⁻.

Dans les composés de la présente invention, les paires d'ions présentent une très forte dissociation, ce qui permet l'expression des propriétés catalytiques intrinsèques du cation M^{m+}, dont les orbitales actives sont facilement exposées aux substrats de la réaction, ceci dans des milieux variés. La plupart des réactions importantes de la chimie organique peuvent être ainsi effectuées dans des conditions peu contraignantes, avec d'excellents rendements et la facilité de séparer le catalyseur du milieu réactionnel. La mise en évidence d'induction asymétrique par l'utilisation d'un composé ionique selon l'invention qui portent un groupement chiral est particulièrement importante de part sa généralité et sa facilité de mise en oeuvre. La présente invention a par conséquent comme autre objet l'utilisation des composés de l'invention comme catalyseurs dans les réactions de Friedel-Crafts, les réactions de Diels et Alder, les réactions d'aldolisation, les additions de Michael, les réactions d'allylation, les réactions de couplage pinacolique, les réactions de glycosilation, les réactions d'ouvertures de cycles des oxétanes, les réactions de méthathèse des alcènes, les polymérisations de type Ziegler-Natta, les polymérisations du type méthathèse par ouverture de cycle et les polymérisations du type méthathèse de diènes acycliques. Les composés ioniques de l'invention préférés pour une utilisation en tant que catalyseur pour les réactions ci-dessus sont ceux dans lesquels le cation est choisi parmi le lithium, le magnésium, le cuivre, le zinc, l'étain, les métaux trivalents, y compris les terres rares, les platinoïdes, et leurs couples organométalliques, en particulier les métallocènes.

Les composés de l'invention peuvent également être utilisés comme solvant pour effectuer des réactions chimiques, photochimiques, électrochimiques, photoélectrochimiques. Pour cette utilisation particulière, on préfère les composés ioniques dans lesquels le cation est un imidazolium, un triazolium, un pyridinium ou un 4-diméthylamino-pyridinium, ledit cation portant éventuellement un substituant sur les atomes de carbone du cycle. Les composés étant utilisés sous leur forme liquide, on préfère tout spécialement ceux qui ont un point de fusion inférieur à 150°C, plus particulièrement inférieur à 100°C.

Les inventeurs ont également trouvé que la charge anionique portée par le groupement anionique exerçait un effet stabilisant sur les conducteurs électroniques de type polymères conjugués, et que l'utilisation d'un composé dans lequel l'un des substituants comprenait une longue chaîne alkyle permettait, de rendre ces polymères solubles dans les solvants organiques usuels même à l'état dopé. Le greffage de ces charges sur le polymère lui-même donne des polymères dont la charge globale est cationique, solubles dans les solvants organiques et présentant, outre leur stabilité, des propriétés d'anticorrosion vis-à-vis des métaux, l'aluminium et les métaux ferreux. La présente invention a aussi pour objet des matériaux à conduction électronique comprenant un composé ionique de la présente invention dans lequel la partie cationique est un polycation constitué par un polymère conjugué dopé "p". Les composés ioniques préférés pour cette application sont ceux dans lesquels l'un des substituants R_{A}, R_{B}, R_{C}, R_{D} ou Z contient au moins une chaîne alkyle ayant de 6 à 20 atomes de carbone.

Les colorants de type cationique (cyanines) sont utilisés de plus en plus fréquemment comme sensibilisateurs de films photographiques, pour le stockage optique d'information (disques optiques accessibles en écriture), pour les lasers. La tendance de ces molécules conjuguées à s'empiler lorsqu'elles sont en phases solides limite leur utilisation, par suite des variations des propriétés optiques par rapport à la molécule isolée. L'utilisation de composés ioniques de l'invention pour la fabrication de colorants cationiques dont les contre ions, éventuellement fixés à cette même molécule, correspondent aux fonctionnalités de l'invention, permet de réduire les phénomènes d'agrégation, y compris dans des matrices solides polymères et de stabiliser ces colorants. La présente invention a pour autre objet une composition de colorant cationique, caractérisée en ce qu'elle contient un composé ionique selon l'invention. Des composés ioniques particulièrement préférés pour cette application sont ceux dans lesquels la ou les charges négatives du groupement anionique sont soit fixées à la molécule de colorant, soit elles constituent le contre-ion des charges positives du colorant. D'autres composés préférés pour cette application sont ceux dans lesquels le radical Z est un radical bivalent comprenant au moins un groupement cationique -(W=W)ₙ-W⁺-, dans lesquels W représente un atome d'azote ou un groupement -C(R)- (R étant un radical organique) et 0≤n≤5.

La présente invention est illustrée par les exemples suivants auxquels elle n'est toutefois pas limitée.

### Exemple 1

A 9,91 g (100 mmoles) d'isocyanate de butyle C₄H₉NCO dans 30 ml de dichlorométhane anhydre, on a ajouté goutte à goutte pendant 30 min, 5,91 g (100 mmoles) de propylamine C₃H₇NH₂ diluée dans 20 ml de dichlorométhane. Après 2 heures, on a ajouté 14,1 g (100 mmoles) de chlorure de malonyle ClCOCH₂COCl dans 100 ml de dichlorométhane anhydre à 0°C sous argon. On a observé un dégagement d'acide chlorhydrique concomitant à la réaction de cyclisation. Après 48 heures, la solution a été évaporée et l'on a récupéré quantitativement après séchage l'acide 1-propyl-3-dibutyl-barbiturique.

A 11,3 g (50 mmoles) de ce composé en solution dans 60 ml de tétrahydrofurane (THF) anhydre, à -20°C et sous agitation mécanique, on a ajouté 11,22 g (100 mmoles) de 1,4-diazabicyclo[2.2.2]octane (DABCO), puis goutte à goutte 8,43 g (100 mmoles) de chlorure de trifluorométhanesulfonyle CF₃SO₂Cl. Après 24 heures, le milieu réactionnel a été agité pendant 4 heures avec 2,12 g (50 mmoles) de chlorure de lithium anhydre. Après filtration pour éliminer le précipité d'hydrochlorure de DABCO, le solvant a été évaporé. On a récupéré quantitativement après séchage le sel de lithium de l'acide 5-trifluorométhanesulfonyl-1-propyl-3-butyl-barbiturique, ayant une pureté déterminée par RMN du proton supérieure à 97%.

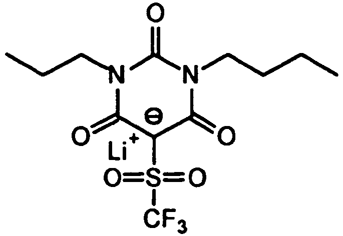

De la même manière, on a obtenu l'acide 5-trifluorométhanesulfonyl-1-allyl-3-butyl-barbiturique, en remplaçant la propylamine par l'allylamine.

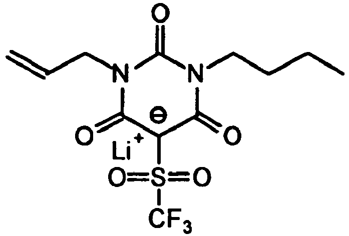

Ces sels sont solubles dans la plupart des solvants organiques usuels (tétrahydrofurane, acétonitrile, diméthylformamide, acétate d'éthyle, glymes,...) et dans les polymères solvatants aprotiques comme le polyoxyde d'éthylène. Dans ce dernier solvant à une concentration O/Li de 12/1, ils présentent une conductivité ionique supérieure à 10⁻⁴. S.cm⁻¹ à une température de 60°C.

### Exemple 2

On a ajouté goutte à goutte durant 1 heure, 134,96 g (1 mole) de chlorure de sulfuryle ClSO₂Cl dilués dans 100 ml de dichlorométhane anhydre, à une solution de 146,23 g (1 mole) de butylamine C₄H₉NH₂ (2 moles) dans 500 ml de THF anhydre à 0°C. La réaction a été poursuivie pendant 2 heures à 0°C, puis pendant 3 heures à l'ambiante. Après filtration du milieu réactionnel pour éliminer l'hydrochlorure de butylamine formé, le solvant a été évaporé à l'aide d'un évaporateur rotatif. Le produit obtenu a été recristallisé dans 200 ml de méthanol, et on a récupéré après filtration et séchage 151 g de N,N'-dibutylsulfamide C₄H₉NHSO₂NHC₄H₉ (rendement 72%) ayant une pureté caractérisée par RMN du proton supérieure à 99%. A 20, 83 g (100 mmoles) de N,N'-dibutylsulfamide et 10,41 g (100 mmoles) d'acide malonique dans 100 ml d'acétonitrile anhydre à 0°C, on a ajouté 41,27 g de 1,3-dicyclohexylcarbodiimide (200 meq). Après 2 heures à 0°C et 24 heures à température ambiante, le milieu réactionnel a été filtré pour éliminer la 1,3-dicyclohexylurée formée, puis le solvant a été évaporé. Le produit obtenu a été recristallisé dans 50 ml de méthanol contenant 9,81 g (100 meq) d'acétate de potassium. Après filtration et séchage, on a récupéré 21,7 g du sel de potassium de l'acide 1,3-dibutyl-2-sulfonyl-barbiturique (rendement 69%) avec une pureté caractérisée par RMN du proton supérieure à 98%.

On a préparé l'acide 1,3-dibutyl-2-sulfonyl-barbiturique par extraction à l'éther du sel de potassium dans de l'eau à un pH inférieur à 2. Puis, par une procédure similaire à celle décrite dans l'exemple 1, on a obtenu le sel de lithium de l'acide 5-trifluorométhanesulfonyl-1,3-dibutyl-2-sulfonylbarbiturique et le sel de lithium de l'acide 5-perfluorobutanesulfonyl-1,3-dibutyl-2-sulfonyl-barbiturique en remplaçant le chlorure de trifluorométhanesulfonyle par le fluorure de perfluorobutanesulfonyle.

On a obtenu les sels de potassium en recristallisant les sels de lithium dans une solution saturée de chlorure de potassium.

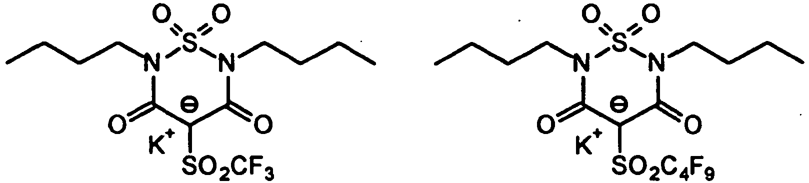

Ces sels sont solubles dans la plupart des solvants organiques usuels (tétrahydrofurane, acétonitrile, diméthylformamide, acétate d'éthyle, glymes,...) et dans les polymères solvatants aprotiques comme le polyoxyde d'éthylène. Dans ce dernier solvant à une concentration O/Li de 12/1, ils présentent une conductivité ionique supérieure à 2.10⁻⁴ S.cm⁻¹ à une température de 60°C. Les sels de lithium, à une concentration aussi faible que 0,1 g/l dans l'eau, abaissent la tension superficielle à une valeur inférieure à 25 mN/m.

### Exemple 3

On a fait réagir 16,02 g (100 mmoles) de malonate d'éthyle C₂H₅OOCCH₂COOC₂H₅ et 11,82 g (200 mmoles) de propylamine C₃H₇NH₂. Après avoir agité la solution pendant 24 heures, on a obtenu une pâte épaisse qui, après séchage, a donné quantitativement la dipropylamide de malonate C₃H₇NHOCCH₂CONHC₃H₇ caractérisée par RMN du proton. On a alors ajouté goutte à goutte 6,75 g (50 mmoles) de chlorure de sulfuryle ClSO₂Cl dilués dans 20 ml de dichlorométhane anhydre à 9,31 g de dipropylamide de malonate (50 meq) en solution dans 30 ml de pyridine à 0°C. La réaction a été menée pendant 2 heures à température ambiante, puis pendant 48 heures à température ambiante et le solvant a été évaporé. Le produit obtenu a été repris dans 30 ml d'eau acidifiée par 13 ml d'acide chlorhydrique 4 M, puis extrait à deux reprises par 25 ml d'éther. Après séchage de la phase organique par le sulfate de magnésium, évaporation de l'éther et séchage du résidu, on a récupéré 11, 67 g (94% de rendement) de l'acide 1,3-dipropyl-2-sulfonyl-barbiturique ayant une pureté caractérisée par RMN du proton supérieure à 97%.

On a obtenu le sel de potassium en traitant l'acide par le carbonate de potassium dans l'eau.

### Exemple 4

10,12 g (100 mmoles) de l'éther vinylique du 3-amino-1-propanol CH₂=CHO(CH₂)₃NH₂ dans 30 ml de THF anhydre ont été traités par 9,91 g (50 mmoles) de 1,1'-sulfonyldiimidazole, préparé par action de l'imidazole sur le chlorure de sulfuryle. Après 48 heures, le milieu réactionnel a été versé dans un ballon contenant 8,01 g (50 mmoles) de malonate d'éthyle C₂H₅O-OCCH₂CO-OC₂H₅ et 8,1 g (75 mmoles) de méthoxyde de sodium CH₃ONa. Après 96 heures à température ambiante, les solvants ont été évaporés et le produit repris dans 30 ml de méthanol. Après addition de 4,91 g (50 mmoles) d'acétate de potassium (50 meq), il s'est formé un précipité qui a été récupéré par filtration sur un verre fritté de porosité N°3, puis lavé à deux reprises par 5 ml d'eau froide. On a obtenu après séchage 11,61 g du sel de potassium de l'acide 1,3-vinyloxypropyl-2-sulfonyl-barbiturique (69% de rendement) caractérisé par RMN du proton.

A 3,7 g (10 mmoles) de ce composé en solution dans 20 ml de THF à -20°C, on a ajouté 2,01 g (10 mmoles) de 1-(trifluorométhanesulfonyl)imidazole (commercialisé par Fluka). La réaction s'est poursuivie pendant 4 heures à - 20°C, puis 48 heures à température ambiante. Le solvant a alors été évaporé et le solide résiduel a été lavé par le dichlorométhane pour éliminer l'imidazole formé au cours de la réaction. On a obtenu le composé suivant :

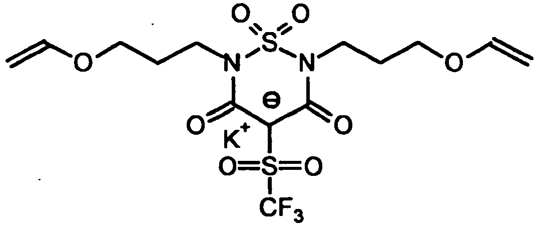

D'autre part, 3,7 g (10 mmoles) du sel de potassium de l'acide 1,3-vinyloxypropyl-2-sulfonyl-barbiturique ont été traités par 1,96 g (10 mmoles) de trifluoroacétate de 2,2,2-trifluoroéthyle dans 15 ml de THF anhydre. Après 24 heures, la solution obtenue a été évaporée et l'on a récupéré quantitativement après séchage le sel de potassium de l'acide 5-trifluoroacétyl-1,3-vinyloxypropyl-2-sulfonyl-barbiturique.

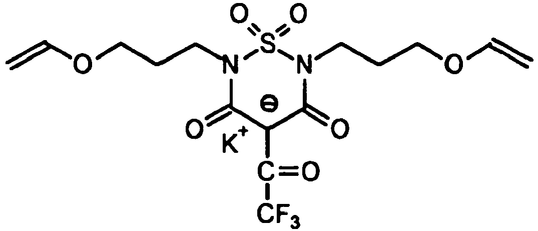

Les homopolymères de ces sels préparés par une polymérisation dans l'acétonitrile anhydre, initiée par voie cationique par la bis(trifluorométhanesulfonyl)imide, présentent une conductivité à une concentration de 0,8 M dans un mélange de diméthylcarbonate et de carbonate d'éthylène (2:1) supérieure à 10⁻³ S.cm⁻¹ à 30°C. De plus, ces homopolymères sont solubles dans la plupart des solvants organiques usuels (tétrahydrofurane, acétonitrile, diméthylformamide, acétate d'éthyle, glymes,...) et dans les polymères solvatants aprotiques comme le poly (oxyde d'éthylène).

### Exemple 5

Dans 20 ml de THF anhydre à -20°C, on a ajouté 29,26 g (40 mmoles) de butylamine puis goutte à goutte 23,7 g (20 mmoles) de chlorofluorosulfone FSO₂Cl. Après une heure, on a ajouté 19,81 g de 2,2,2-trifluoroéthylamine CF₃CH₂NH₂ (20 mmoles) et 2 ml de pyridine, et la réaction s'est poursuivie pendant 2 heures à -20°C puis 48 heures à l'ambiante. Le milieu réactionnel a été filtré puis le solvant évaporé. Après recristallisation du résidu dans le méthanol, on a récupéré la N-2,2,2-trifluoroéthyl-N'-butyl-sulfamide.

Par un procédé similaire, on a préparé la N-2,2,3,3,3-pentafluoropropyl-N'-butyl-sulfamide, en remplaçant la 2,2,2-trifluoroéthylamine par la 2,2,3,3,3-pentafluoropropylamine, et la N-2,2,3,3,4,4,4-heptafluorobutyl-N'-butyl-sulfamide en remplaçant la 2,2,2-trifluoroéthylamine par la 2,2,3,3,4,4,4-heptafluorobutylamine.

On a obtenu les acides barbituriques et les sels de potassium correspondants à ces trois composés suivant une procédure similaire à celle décrite dans l'exemple 2.

On a obtenu les sels de potassium des acides portant un groupement trifluorométhanesulfonyle en C-5 par une procédure similaire à celle décrite dans l'exemple 4.

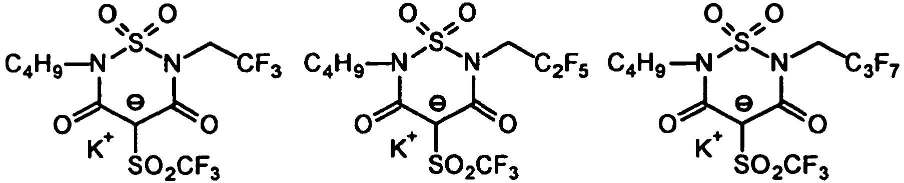

Ces sels sont solubles dans la plupart des solvants organiques usuels (tétrahydrofurane, acétonitrile, diméthylformamide, acétate d'éthyle, glymes,...) et dans les polymères solvatants aprotiques.

### Exemple 6

Dans 200 ml de dichlorométhane anhydre à -20°C, sous agitation mécanique et sous argon, contenant 20,81 g (200 mmoles) d'acide malonique HOOCCH₂COOH et 41,63 g (200 mmoles) d'hexafluoroacétone trihydrate CF₃COCF₃·3H₂O (commercialisé par Aldrich), on a ajouté lentement 95,17 g (800 mmoles) de chlorure de thionyle SOCl₂ dilué par 100 ml de dichlorométhane anhydre. Lorsque l'addition a été terminée (environ 2 heures), la réaction s'est poursuivie pendant 24 heures à -20°C puis 24 heures à l'ambiante. Le solvant a alors été évaporé et le résidu repris dans 100 ml d'eau. On a alors ajouté par portions 15,2 g (110 mmoles) de carbonate de potassium anhydre K₂CO₃, puis on a recristallisé le précipité obtenu après avoir ajouté 14,91 g (200 mmoles) de chlorure de potassium anhydre KCl. Après filtration et séchage, on a récupéré 35,8 g (71% de rendement) du sel de potassium de la 2,2-trifluorométhyl-1,3-dioxolane-4,6-dione (I), ayant une pureté déterminé par RMN du proton supérieure à 98%.

On a obtenu le sel de lithium par échange ionique (métathèse) avec le chlorure de lithium dans le THF.

### Exemple 7

5,38 g (20 mmoles) de chlorure de l'acide dodécylsulfonique C₁₂H₂₅SO₂Cl (commercialisé par Lancaster) dans 30 ml de THF anhydre et 10 ml de pyridine ont été ajoutés à 6,29 g (20 mmoles) du sel de potassium de l'acide 1,3-dibutyl-2-sulfonyl-barbiturique, préparé dans des conditions analogues à celles décrites dans l'exemple 2. Après 24 heures, la solution légèrement colorée a été filtrée pour éliminer le précipité de chlorure de potassium, puis mise en contact avec 800 mg de carbonate de lithium Li₂CO₃ et 4 g de charbon actif. Le mélange a été agité pendant 24 heures, puis le carbonate en excès et le charbon actif ont été éliminés en filtrant la solution et le solvant a été évaporé. On a obtenu quantitativement 10,9 g du sel de lithium de l'acide 5-dodécylsulfonyl-1,3-dibutyl-2-sulfonylbarbiturique caractérisé par RMN du proton : 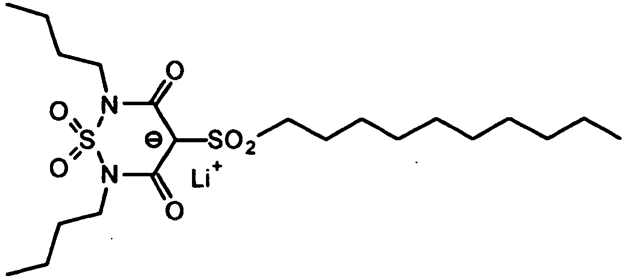 qui possède des propriétés tensio-actives marquées, y compris dans les solvants aprotiques et plus particulièrement dans les polymères solvatants aprotiques.

### Exemple 8

Dans un premier temps, on a préparé le disel de sodium de l'acide 2,2'-Azinobis(3-éthylbenzothiazoline-6-sulfonique) à partir de son disel d'ammonium (commercialisé par Aldrich), en le traitant par une solution titrée d'hydroxyde de sodium. Après évaporation et séchage, on a récupéré quantitativement le disel de sodium. A 1,12 gr de ce dernier (2 mmoles) dans 10 ml d'acétonitrile anhydre, on a ajouté lentement 508 mg de chlorure d'oxalyle ClCOCOCl (4 mmoles) en solution dans 1 ml de dichlorométhane anhydre. Après 4 heures sous agitation, on a ajouté 4 ml de pyridine anhydre et 1,47 g (4 mmoles) du sel de potassium de l'acide 1,3-(2,2,2-trifluoroéthyl)-2-sulfonyl-barbiturique, obtenu à l'exemple 3. Après 24 heures, l'acétonitrile a été évaporé et le résidu repris dans 10 ml d'eau. Après avoir ajouté 1,29 g de bromure de tétrabutylammonium, le précipité obtenu à été extrait au dichlorométhane. Après séchage de la phase organique par du sulfate de magnésium, évaporation du solvant et séchage, on a obtenu le composé suivant :

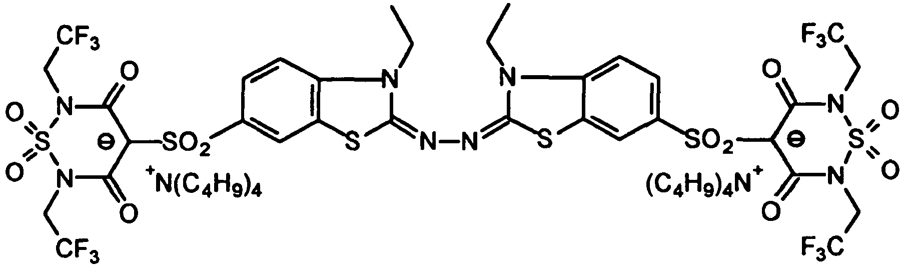

Ce composé donne par oxydation un radical et un biradical qui sont des zwitterions stables et il permet de faire des catalyses d'oxydation entre une phase aqueuse oxygénée et une phase organique non miscible contenant les espèces à oxyder.

### Exemple 9

A 4, 9 g du sel de sodium de l'acide Nickel (II) phtalocyaninetétrasulfonique (5 mmoles, commercialisé par Aldrich) dans 40 ml de diméthylformamide (DMF) anhydre, on a ajouté par portions 3,2 g de chlorure de (chlorométhylène)diméthylammonium [(CH₃)₂N=CHCl]⁺,Cl⁻ (25 mmoles, commercialisé par Aldrich). Après 4 heures sous agitation, le milieu réactionnel a été centrifugé, le liquide surnageant éliminé puis le produit décanté repris dans 40 ml de DMF anhydre. On a alors ajouté 2 ml de pyridine et 7,81 g (20 mmoles) du sel de potassium de l'acide 1-(2,2,3,3,3-pentafluoropropyl)-3-butyl-2-sulfonyl-barbiturique obtenu à l'exemple 5. Après 48 heures sous agitation, le milieu réactionnel a été agité 24 heures avec 2 g de carbonate de potassium Li₂CO₃. Après évaporation de la DMF et séchage, on a obtenu le composé suivant : 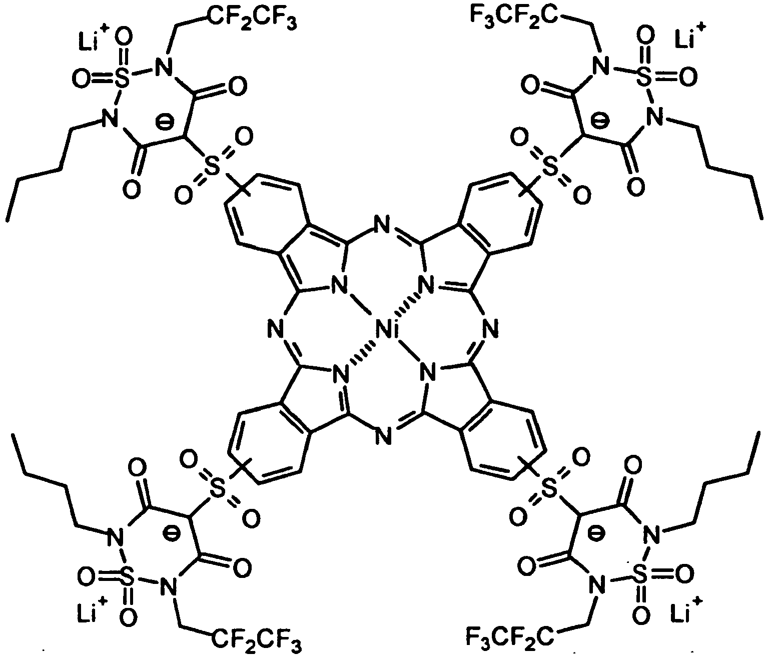 soluble dans la plupart des solvants organiques usuels (tétrahydrofurane, acétonitrile, diméthylformamide, acétate d'éthyle, glymes,...) et dans les polymères polaires. Il présente une absorption importante dans le visible. Sous la forme de tétrasel de tétrabutylammonium, il est également soluble dans les solvants peu polaires comme le dichlorométhane ou le chlorure de méthylène ainsi que dans les matrices polymères peu polaires comme le polyméthacrylate de méthyle.

Le greffage de l'acide 1-(2,2,3,3,3-pentafluoropropyl)-3-butyl-2-sulfonyl-barbiturique permet également de diminuer nettement l'agrégation des molécules de ce colorant cationique entre elles, ce phénomène d'agrégation amenant un élargissement des bandes d'absorption optiques préjudiciable à la précision de fonctionnement des systèmes utilisant ses colorants en particulier les disques optiques de stockage d'informations.

### Exemple 10

On a introduit dans un ballon tricol équipé d'un réfrigérant, d'une agitation mécanique et d'une entrée de gaz neutre (Argon), 9,5 g d'un copolymère de diméthylsiloxane et de (hydrogéno)(méthyl)-siloxane (HMS 301 25% SiH, M_{w} 1900, commercialisé par Gelest Inc., Tullytown, PA, USA) en solution dans du tétrahydrofurane. On a ensuite ajouté 14,03 g du sel de lithium de l'acide 5-trifluoroacétyl-3-allyl-1-butyl-barbiturique, obtenu à l'exemple 1, et 70 mg d'acide chloroplatinique H₂PtCl₆. Le mélange a été chauffé au reflux pendant 4 heures. Le polymère a ensuite été reprécipité dans l'éther, redissous dans le THF et de nouveau reprécipité dans l'éther. On a obtenu le polymère suivant : 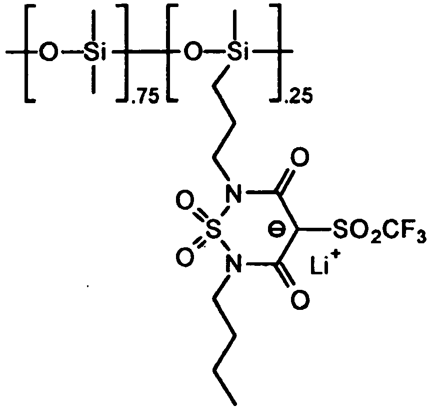 soluble dans la plupart des solvants organiques, y compris à des teneurs > 2% dans les huiles ou les matériaux siliconés, auxquels il confère des propriétés antistatiques.

### Exemple 11

5,97 g (10 mmoles) du sel de potassium de l'acide 5-perfluorobutanesulfonyl-1,3-dibutyl-2-sulfonyl-barbiturique, préparé selon l'exemple 5 et 3,17 g (10 mmoles) de chlorure de diphényliodonium (C₆H₅)₂ICl et, ont été agités ensemble durant 24 heures dans l'eau. Par extraction de la phase aqueuse par du dichlorométhane, on a récupéré le composé suivant :

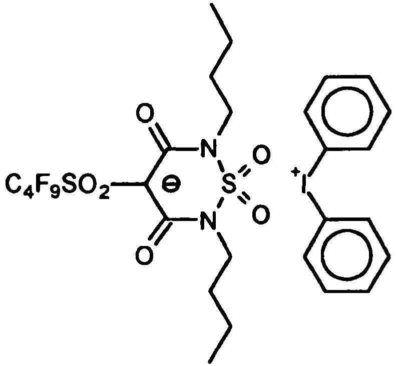

Ce sel est particulièrement actif comme photoinitiateur de polymérisation cationique, en particulier pour les di vinyléthers tels que le divinyléther du triéthylène glycol (DVE-3). Cet amorceur possède une solubilité et une activité supérieure à celle du même sel de sulfonium associé aux l'anions PF₆⁻ ou SbF₆⁻.

### Exemple 12

2,22 g (5 mmoles) de tétra-fluoroborate de tétrakis-(acétonitrile)palladium(II) (CH₃CN)₄Pd(BF₄)₂ (commercialisé par Aldrich), dans 30 ml de tétrahydrofurane, ont été traités par 5,97 g (10 mmoles) du sel de potassium de l'acide 5-perfluorobutanesulfonyl-1,3-dibutyl-2-sulfonyl-barbiturique obtenu à l'exemple 2. Après 24 heures sous agitation, le milieu réactionnel a été filtré pour éliminer le précipité de tétrafluoroborate de potassium KBF₄, puis le solvant évaporé. On a obtenu quantitativement le composé suivant :

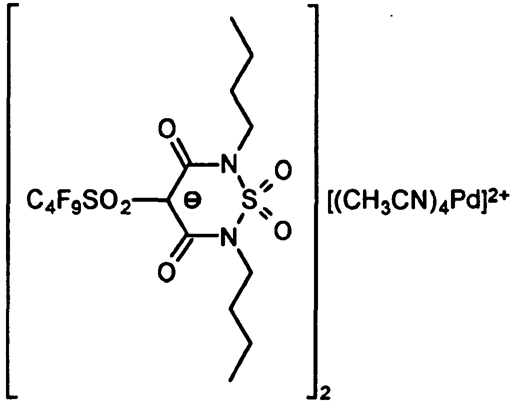

Ce sel est un catalyseur de la polymérisation vinylique du norbornène même dans les solvants peu polaires comme le dichlorométhane.

### Exemple 13

### Catalyse d'une condensation aldolique

On a obtenu le sel de scandium de l'acide 5-trifluoro-méthanesulfonyl-1,3-dibutyl-2-sulfonyl-barbiturique, en traitant le sel de potassium, obtenu à l'exemple 2, par une quantité stoechiométrique de tétrafluoroborate de scandium Sc(BF₄)₃ dans l'acétonitrile. Après filtration pour éliminer le précipité de tétrafluoroborate de potassium KBF₄ et évaporation du solvant, on a récupéré quantitativement le sel de scandium du dibutylaminosulfonylmalononitrile (Sc(DBTFSB)₃).

L'effet catalytique de ce sel vis à vis d'une condensation aldolique a été évalué de la manière suivante. A une solution contenant 507 mg (0,4 mmoles) du sel de scandium de l'acide 5-trifluorométhylsulfonyl-1,3-dibutyl-2-sulfonyl-barbiturique (10% en mole) dans 15 ml de dichlorométhane, on a ajouté un mélange de 1,05 g (6 mmoles) de 1-ène-2-méthyl-1-silylacétal-1-méthoxypropène (CH₃)₂C=C(OSiMe₃)OMe et de 420 mg (4 mmoles) de benzaldéhyde dans 10 ml de dichlorométhane. Après 16 heures sous agitation à l'ambiante, on a ajouté de l'eau et le produit a été extrait avec du dichlorométhane. La phase organique a été lavée par trois fractions de 100 ml d'eau, puis le dichlorométhane évaporé. Le résidu a alors été traité par un mélange tétrahydrofurane/HCl 1 M (20:1) pendant 0,5 heures à 0°C. Après avoir dilué avec de l'hexane, on a ajouté une solution saturée d'hydrogénocarbonate de sodium, puis le produit a été extrait au dichlorométhane. La phase organique a été lavée avec une solution saturée de chlorure de sodium, puis séchée avec du sulfate de sodium. Après évaporation des solvants, le produit brut a été chromatographié sur un gel de silice. On a obtenu le méthyl-3-hydroxy-2,2-diméthyl-phénylpropionate avec un rendement de 91% en accord avec les résultats obtenus avec le sel d'ytterbium du triflate de l'art antérieur.

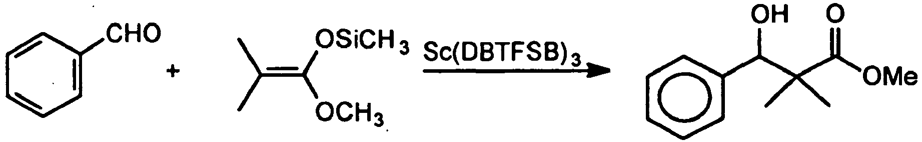

La même réaction a été effectuée avec une quantité de catalyseur diminuée d'un facteur proche de cinq, sans diminution du rendement en composé méthyl-3-hydroxy-2,2-diméthyl-phénylpropionate. Ce résultat est du à la meilleure solubilité dans le dichlorométhane du sel de scandium de l'acide 5-trifluorométhanesulfonyl-1,3-dibutyl-2-sulfonyl-barbiturique, comparativement au sel d'ytterbium du triflate, utilisé dans l'art antérieur.

### Exemple 14

### Catalyse d'une addition de Michael

L'effet catalytique du sel de scandium de l'acide 5-trifluorométhanesulfonyl-1,3-dibutyl-2-sulfonyl-barbiturique, obtenu à l'exemple 13, vis à vis d'une addition de Michael a été évalué de la manière suivante. A une solution de 507 mg (0,4 mmoles) du sel de scandium du dibutylaminosulfonylmalononitrile (10% en mole) dans 15 ml de dichlorométhane, on a ajouté un mélange de 840 mg (4 mmoles) de chalcone et de 1,05 g (6 mmoles) de 1-ène-2-méthyl-1-silylacétal-1-méthoxypropène (CH₃)₂C=C(OSiMe₃)OMe dans 10 ml de dichlorométhane. Après 12 heures sous agitation à l'ambiante, on a ajouté de l'eau et le produit a été extrait avec du dichlorométhane. La phase organique a été lavée par trois fractions de 100 ml d'eau, puis le dichlorométhane a été évaporé. Le résidu a alors été traité par un mélange tétrahydrofurane/HCl 1 M (20:1) pendant 0,5 heure à 0°C. Après avoir dilué avec de l'hexane, on a ajouté une solution saturée d'hydrogénocarbonate de sodium, puis le produit a été extrait au dichlorométhane. La phase organique a été lavée avec une solution saturée de chlorure de sodium, puis séchée avec du sulfate de sodium. Après évaporation des solvants, le produit brut a été chromatographié sur un gel de silice. On a obtenu le composé 1,5-dicarbonylé avec un rendement de 85% en accord avec les résultats obtenus avec le sel d'ytterbium du triflate de l'art antérieur.

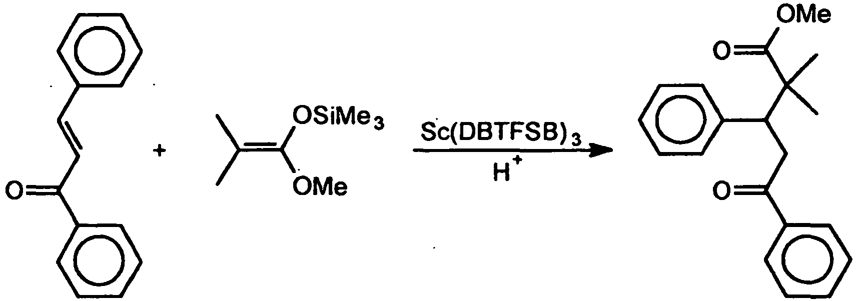

La même réaction a été effectuée avec une quantité de catalyseur diminuée d'un facteur proche de cinq, sans diminution du rendement en composé 1,5-dicarbonylé. Ce résultat est du à la meilleure solubilité dans le dichlorométhane du sel de scandium de l'acide 5-trifluorométhanesulfonyl-1,3-dibutyl-2-sulfonylbarbiturique, comparativement au sel d'ytterbium du triflate.

### Exemple 15

Catalyse d'une réaction d'acylation Friedel-Crafts.

L'effet catalytique du sel de scandium de l'acide 5-trifluorométhanesulfonyl-1,3-dibutyl-2-sulfonyl-barbiturique, obtenu à l'exemple 13, vis à vis d'une réaction d'acylation Friedel-Crafts, a été évalué de la manière suivante. Dans 40 ml de nitrométhane anhydre, on a ajouté 887 mg (0,7 moles) du sel de scandium de l'acide 5-trifluorométhylsulfonyl-1,3-dibutyl-2-sulfonyl-barbiturique, puis 1,08 g (10 mmoles) d'anisole et 2,04 g (20 mmoles) d'anhydride acétique. Après agitation pendant 10 min à 21°C, le milieu réactionnel a été dilué par 50 ml d'éther et la réaction inhibée par 100 ml d'une solution saturée d'hydrogénocarbonate de sodium NaHCO₃. Après filtration sur de la Celite, la solution a été extraite par trois fractions de 50 ml d'éther, puis la phase éthérée recueillie a été lavée par une solution saturée de chlorure de potassium. Après séchage par du sulfate de magnésium de la phase éthérée et évaporation, on a récupéré 1,46 g de p-méthoxyacétophénone (95% de rendement) avec une pureté caractérisée par RMN du proton supérieure à 99%.

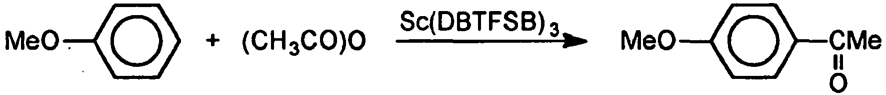

### Exemple 16

L'effet catalytique du sel de scandium de l'acide 5-trifluorométhanesulfonyl-1,3-dibutyl-2-sulfonyl-barbiturique, obtenu à l'exemple 13, vis à vis d'une réaction de Diels-Alder, a été évalué en effectuant la réaction suivante.

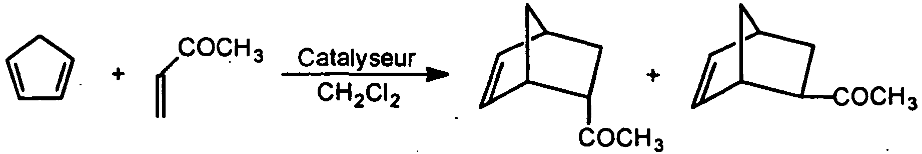

A une solution dans 10 ml de dichlorométhane de 651 mg de cyclopentadiène (10 mmoles) fraîchement distillé et de 701 mg de méthylvinylcétone, on a ajouté 200 µmoles du sel de scandium de l'acide 5-trifluorométhylsulfonyl-1,3-dibutyl-2-sulfonyl-barbiturique. Après 24 heures à l'ambiante, le milieu réactionnel a été filtré pour éliminer le catalyseur en suspension. On a obtenu un rendement de réaction, déterminé par chromatographie en phase gazeuse, supérieur à 90%.

### Exemple 17

Le sel de lithium de l'acide 5-trifluorométhylsulfonyl-1,3-dibutyl-2-sulfonyl-barbiturique, obtenu à l'exemple 2, et le sel de lithium de l'acide 5-cyano-2,2-trifluorométhyl-1,3-dioxolane-4,6-dione, obtenu à l'exemple 6, ont été testés dans des générateurs électrochimiques de technologie lithium-polymère.

Pour chaque sel, on a réalisé une batterie superposant les couches suivantes :
- un collecteur de courant en acier inoxydable ayant une épaisseur de 2 mm ;
- une cathode constituée par une pastille d'un film de matériau composite ayant une épaisseur de 89 µm et constitué par du dioxyde de vanadium (45% en volume), du noir de Shawinigan (5% en volume) et un polyoxyde d'éthylène de masse M_{w} = 3.10⁵ (50% en volume) ;
- un électrolyte constitué par une pastille d'un film de polyoxyde d'éthylène de masse M_{w} = 5.10⁶ contenant un des deux sels de lithium à une concentration O/Li = 15/1 ;
- une anode constituée par une feuille de lithium métallique ayant une épaisseur de 50 µm ;
- un collecteur de courant analogue au collecteur précité.

Les pastilles constituant les électrodes et l'électrolyte ont été découpées en boîte à gants et empilées dans l'ordre indiqué ci-dessus. Les collecteurs ont ensuite été déposés de part et d'autre de l'empilement obtenu.

On a scellé le tout dans un boîtier de pile bouton, qui permet à la fois de protéger le générateur de l'atmosphère et d'excercer une contrainte mécanique sur les films. La batterie a alors été placée dans une enceinte sous argon installée dans une étuve à une température de 60°C. Elle a ensuite été cyclée entre 1,8 et 3,3 V à un régime de charge et de décharge de C/10 (capacité nominale chargée ou déchargée en 10 heures).

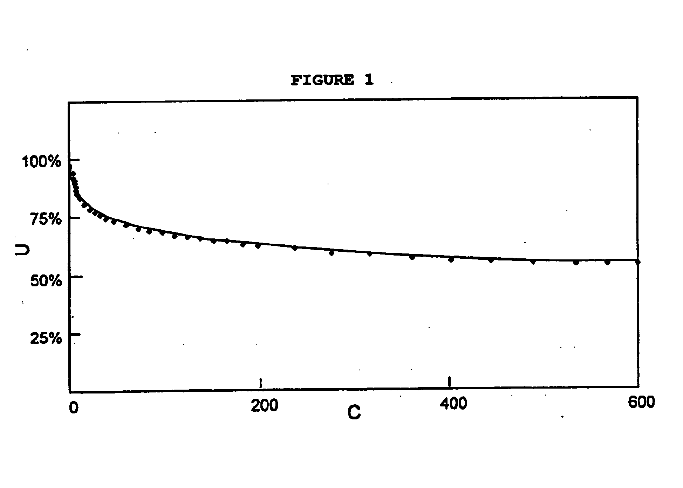

La courbe de cyclage obtenue en utilisant le sel de lithium de l'acide 5-trifluorométhylsulfonyl-1,3-dibutyl-2-sulfonyl-barbiturique est donnée sur la figure 1. Sur cette figure, l'utilisation, U, exprimée en % est donnée en ordonnée, et le nombre de cycles C est donné en abscisse.

On a obtenu un résultat de cyclage similaire avec le sel de lithium de l'acide 5-cyano-2,2-trifluorométhyl-1,3-dioxolane-4,6-dione.

## Revendications

1. Composé ionique comprenant au moins une partie anionique associée à au moins une partie cationique M en nombre suffisant pour assurer la neutralité électronique de l'ensemble, **caractérisé en ce que** M est un hydroxonium, un nitrosonium NO⁺, un ammonium -NH₄⁺, un cation métallique ayant la valence m, un cation organique ayant la valence m ou un cation organométallique ayant la valence m, et **en ce que** la partie anionique est un hétérocycle aromatique répondant à l'une des formules 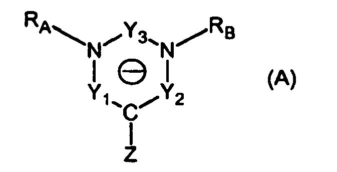 dans lesquelles :
- Y₁ et Y₂ représente un groupement carbonyle :
- Y₃ représente un groupement carbonyle ou un groupement sulfonyle ;
- Z représente un radical électro-attracteur choisi parmi les radicaux R_{E}SO₂ dans lesquels R_{E} représente radical alkyle, alkényle, oxaalkyle, oxaalkényle, azaalkyle, azaalkényle, thiaalkyle, thiaalkényle, aryle, alkylaryle, alkénylaryle, arylalkyle, arylalkényle, un radical alicyclique ou un radical aromatique portant éventuellement au moins une chaîne latérale comprenant un hétéroatome ou comprenant éventuellement au moins un hétéroatome dans le cycle, ledit R_{E} pouvant être halogéné ou perhalogéné ;
- R_{A} et R_{B} représentent, indépendamment l'un de l'autre :
a) un alkyle, un alkényle, un oxa-alkyle, un oxa-alkényle, un aza-alkyle, un aza-alkényle, un thia-alkyle, un thia-alkényle, lesdits radicaux portant éventuellement au moins un groupe aryle ;
b) un aryle portant au moins éventuellement au moins un radical tel que défini en a) ;
c) un radical alicyclique ou un radical aromatique portant éventuellement au moins une chaîne latérale comprenant un hétéroatome ou comprenant éventuellement au moins un hétéroatome dans le cycle ;
d) un radical tel que défini ci-dessus en a), b) et c) et portant en outre des atomes d'halogène, sous la forme halogénée ou perhalogénée.

2. Composé selon la revendication 1, **caractérisé en ce que** le cation organique est un cation onium choisi dans le groupe constitué par les cation R₃O⁺ (oxonium), NR₄⁺ (ammonium), RC (NHR₂)₂⁺ (amidinium), C(NHR₂)₃⁺ (guanidinium), C₅R₆N⁺ (pyridinium), C₃R₅N₂⁺ (imidazolium), C₃R₇N₂⁺ (imidazolinium), C₂R₄N₃⁺ (triazolium), SR₃⁺ (sulfonium), PR₄⁺ (phosphonium), IR₂⁺ (iodonium), (C₆R₅)₃C⁺ (carbonium); les radicaux R représentant de manière indépendante un H ou un radical choisi dans le groupe constitué par :
- les radicaux alkyles, alkényles, oxa-alkyles, oxa-alkényles, aza-alkyles, aza-alkényles, thia-alkyles, thia-alkényles, sila-alkyles, sila-alkényles, aryles, arylalkyles, alkylaryles, alkényl-aryles, les radicaux dialkylamino et les radicaux dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore ;
étant entendu que plusieurs radicaux R peuvent former ensemble des cycles aliphatiques ou aromatiques englobant éventuellement le centre portant la charge cationique.

3. Composé selon la revendication 2, **caractérisé en ce que** le cation onium fait partie du radical Z.

4. Composé selon la revendication 2, **caractérisé en ce que** le cation onium fait partie d'une unité récurrente d'un polymère.

5. Composé selon la revendication 2, **caractérisé en ce que** le cation onium comprend un groupe hétérocyclique cationique à caractère aromatique, comportant au moins un atome d'azote alkylé dans le cycle.

6. Composé selon la revendication 5, **caractérisé en ce que** le cation onium comprend un groupe imidazolium, un groupe triazolium, un groupe pyridinium, un groupe 4-diméthylamino-pyridinium, lesdits groupes portant éventuellement un substituant sur les atomes de carbone du cycle.

7. Composé selon la revendication 2, **caractérisé en ce que** le cation onium est un groupement possédant une liaison -N=N-, -N=N⁺, un groupement sulfonium, un groupement iodonium, ou un cation arène-ferrocénium substitué ou non, éventuellement incorporé dans une trame polymérique.

8. Composé selon la revendication 7, **caractérisé en ce que** le cation onium est un cation diaryliodonium, un cation dialkylaryliodonium, un cation triarylsulfonium, un cation trialkylaryle sulfonium, ou un cation phénacyl-dialkyl sulfonium substitué ou non.

9. Composé selon la revendication 2, **caractérisé en ce que** le cation onium fait partie d'un polymère.

10. Composé selon la revendication 2, **caractérisé en ce que** le cation onium comprend un groupement 2,2'[Azobis(2-2'-imidazolinio-2-yl)propane]²⁺ ou 2,2'-Azobis(2-amidiniopropane)²⁺.

11. Composé selon la revendication 1, **caractérisé en ce que** le cation est un cation métallique choisi dans le groupe constitué par les cations de métaux alcalins, les cations de métaux alcalino-terreux, les cations de métaux de transition et les cations de métaux trivalents.

12. Composé selon la revendication 1, **caractérisé en ce que** le cation est un métallocénium, choisi dans le groupe constitué par les cations dérivés du ferrocène, du titanocène, du zirconocène, les cations indénocénium, les cations arène métallocénium, les cations des métaux de transition complexés par des liguands de type phosphine possédant éventuellement une chiralité et les cations organométalliques possédant un ou plusieurs groupements alkyles ou aryles fixés d'une manière covalente à un atome ou un groupe d'atome, lesdits cations faisant éventuellement partie d'une chaîne polymère.

13. Composé selon l'une quelconque des revendications précédentes, **caractérisé en ce que** les substituants R_{A} et R_{B} sont choisis indépendamment l'un de l'autre parmi les radicaux alkyles et les radicaux alkényles ayant de 1 à 10 atomes de carbone.

14. Composé selon l'une quelconque des revendications précédentes, **caractérisé en ce que** les substituants R_{A} et R_{B} sont choisis indépendamment l'un de l'autre parmi les radicaux alkyles ou alkényles halogénés ou perhalogénés ayant de 1 à 10 atomes de carbone.

15. Composé selon l'une quelconque des revendications précédentes, **caractérisé en ce que** les substituants R_{A} et R_{B} sont choisis indépendamment l'un de l'autre parmi les radicaux oxa-alkyles ou oxa-alkényles ayant de 1 à 10 atomes de carbone.

16. Matériau à conduction ionique comprenant un composé ionique en solution dans un solvant, **caractérisé en ce que** le composé ionique est un composé selon la revendication 1.

17. Générateur électrochimique comprenant une électrode négative et une électrode positive séparées par un électrolyte, **caractérisé en ce que** l'électrolyte est un matériau selon la revendication 16.

18. Matériau à conduction électronique, **caractérisé en ce qu'**il contient un composé selon la revendication 1.

## Claims

1. An ionic compound comprising at least one anionic part associated to at least one cationic part M in sufficient number to ensure an electronic neutrality to the compound, **characterized in that** M is an hydroxonium, a nitrosonium NO⁺, an ammonium -NH₄⁺, a metal cation having a valency m, an organic cation having a valency m or an organometallic cations having a valency m, and **in that** the anionic part is an aromatic heterocycle corresponding to formula 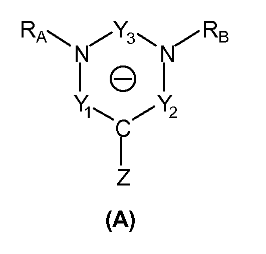 in which:
- Y₁ and Y₂ represent a carbonyl group;
- Y₃ represents a carbonyl group or a sulfonyl group; Z represents an electroattractor radical selected from R_{E}SO₂ groups wherein R_{E} represents an alkyl, alkenyl, oxaalkyl, oxaalkenyl, azaalkyl, azaalkenyl, thiaalkyl, thiaalkenyl, aryl, alkylaryl, alkenylaryl, arylalkyl, arylalkenyl radical, an alicyclic radical or an aromatic radical optionally carrying at least one lateral chain comprising a heteroatom or optionally comprising at least one hetero atom in the cycle, said R_{E} optionally being halogenated or perhalogenated;
- R_{A} and R_{B} represent independently from one another :
a) an alkyl, an alkenyl, an oxa-alkyl, an oxa-alkenyl, an aza-alkyl, an aza-alkenyl, a thia-alkyl, a thia-alkenyl, said radicals carrying at least one aryl group;
b) an aryl carrying at least one radical as defined in a);
c) an alicyclic radical or an aromatic radical optionally carrying at least one lateral chain comprising a heteroatom or optionally comprising at least one heteroatom in the cycle;
d) a radical as defined above in a), b) and c) and additionally carrying halogen atoms, in halogenated or perhalogenated form.

2. A compound according to claim 1, **characterized in that** the organic cation is an onium cation selected from a group consisting of cations R₃O⁺ (oxonium), NR₄⁺ (ammonium), RC(NHR₂)₂⁺ (amidinium), C(NHR₂)₃⁺ (guanidinium), C₅R₆N⁺ (pyridinium), C₃R₅N₂⁺ (imidazolium), C₃R₇N₂⁺ (imidazolinium), C₂R₄N₃⁺ (triazolium), SR₃⁺ (sulfonium), PR₄⁺ (phosphonium), IR₂⁺ (iodonium), (C₆R₅)₃ C⁺ (carbonium), the radicals R independently representing an H or a radical selected from the group consisting of:
- alkyl, alkenyl, oxa-alkyl, oxa-alkenyl, aza-alkyl, aza-alkenyl, thia-alkyl, thia-alkenyl, sila-alkyl, sila-alkenyl, aryl, arylalkyl, alkylaryl, alkenyl-aryl, dialkylamino and dialkylazo radicals;
- cyclic or heterocyclic radicals optionally comprising at least one lateral chain comprising heteroatoms such as oxygen, nitrogen, sulfur;
- cyclic or heterocyclic radicals optionally comprising heteroatoms in the aromatic nucleus;
- groups comprising a plurality of aromatic or heterocyclic nuclei, condensed or non-condensed, optionally containing at least one nitrogen, oxygen, sulfur or phosphorus atom;
with the proviso that a plurality of radicals R may together form aliphatic or aromatic cycles optionally enclosing the center carrying the cationic charge.

3. A compound according to claim 2, **characterized in that** the onium cation is part of the radical Z.

4. A compound cond according to claim 2, **characterized in that** the onium cation is part of a recurring unit of a polymer or is part of a polymer chain.

5. A compound according to claim 2, **characterized in that** the organic cation comprises a cationic heterocycle with aromatic character, having at least one nitrogen atom which is alkylated in the cycle.

6. A compound according to claim 5, **characterized in that** the onium cation comprises an imidazolium group, a triazolium group, a pyridinium group, a 4-dimethyl-amino-pyridinium group, said groups optionally carrying a substituent on the carbon atoms of the cycle.

7. A compound according to claim 2, **characterized in that** the ionium cation est a group having a bond --N=N--, --N=N⁺, a sulfonium group, an iodonium group, or a substituted or non-substituted arene-ferrocenium cation, optionally incorporated in a polymeric network.

8. A compound according to claim 7, **characterized in that** the onium cation is diaryliodonium cation, a dialkylaryliodonium cation, a triarylsulfonium cation, a trialkylaryl sulfonium cation, or a substituted or non-substituted phenacyldialkyl sulfonium cation.

9. A compound according to claim 2, **characterized in that** the onium cation is part of a polymer.

10. A compound according to claim 2, **characterized in that** the onium cation comprises a group 2,2'[Azobis(2-2'-imidazolinio-2-yl)propane]²⁺ or 2,2'-Azobis(2-amidinio-propane)²⁺.

11. A compound ccording to claim 1, **characterized in that** the cation M is a metallic cation selected from the group consisting of cations of alkali metals, cations of alkali earth metals, cations of transition metals, and cations of trivalent metals.

12. A compound according to claim 1, **characterized in that** the cation is a metallocenium, selected from cations derived from ferrocene, titanocene, zirconocene, indenocenium cations, arene metallocenium cations, cations of transition metals complexed with ligands of phosphine type optionally having a chirality and an organometallic cations having a one or more alkyl or aryl groups covalently fixed to an atom or a group of atoms, said cations optionally being part of a polymer chain.

13. A compound according to any of preceding claims, **characterized in that** the substituents R_{A} and R_{B} are selected independently from one another from alkyl radicals and alkenyl radicals having 1 to 10 carbon atoms.

14. A compound according to any of preceding claims, **characterized in that** the substituents R_{A} and R_{B} are selected independently from one another from halogenated alkyl radicals and alkenyl radicals having 1 to 10 carbon atoms.

15. A compound according to any of preceding claims, **characterized in that** the substituents R_{A} and R_{B} are selected independently from one another from oxa-alkyl radicals or oxa- alkenyl radicals having 1 to 10 carbon atoms.

16. An ionically conductive material comprising an ionic compound in solution in a solvent, **characterized in that** the ionic compound is a compound according to claim 1.

17. Electrochemical generator comprising a negative electrode and a positive electrode separated by an electrolyte, **characterized in that** the electrolyte is a material according to claim 16.

18. An electronically conducting material, **characterized in that** is contains a compound according to claim 1.

## Patentansprüche

1. Ionenverbindung, die mindestens einen anionischen Abschnitt umfasst, der mit mindestens einem kationischen Abschnitt M in ausreichender Anzahl verbunden ist, um die elektronische Neutralität der Gruppe zu gewährleisten, **dadurch gekennzeichnet, dass** M ein Hydroxonium, ein Nitrosonium NO⁺, ein Ammonium - NH₄⁺, ein Metallkation mit der Wertigkeit m, ein organisches Kation mit der Wertigkeit m oder ein organometallisches Kation mit der Wertigkeit m ist, und dass der anionische Abschnitt eine aromatische heterocyclische Verbindung ist, die einer der Formeln 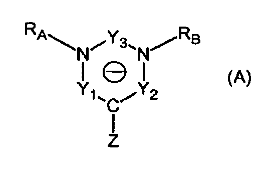 entspricht, wobei:
- Y₁ und Y₂ eine Carbonylgruppe darstellt,
- Y₃ eine Carbonylgruppe oder eine Sulfonylgruppe darstellt,
- Z ein elektronenziehendes Radikal darstellt, das aus den R_{E}SO₂-Radikalen ausgewählt ist, wobei R_{E} Alkyl-, Alkenyl-, Oxaalkyl-, Oxaalkenyl-, Azaalkyl-, Azaalkenyl-Thiaalkyl-, Thiaalkenyl-, Aryle, Alkylaryl-, Alkenylaryl-, Arylalkyl-, Arylalkenylradikal, ein alicyclisches Radikal oder ein aromatisches Radikal darstellt mit eventuell mindestens einer Seitenkette, die ein Heteroatom umfasst oder eventuell mindestens ein Heteroatom in dem Ring umfasst, wobei das R_{E} halogeniert oder perhalogeniert sein kann,
- R_{A} und R_{B} unabhängig voneinander darstellen:
a) ein Alkyl, ein Alkenyl, ein Oxaalkyl, ein Oxaalkenyl, ein Azaalkyl, ein Azaalkenyl, ein Thiaalkyl, ein Thiaalkenyl, wobei die Radikale eventuell mindestens eine Arylgruppe tragen,
b) ein Aryl, das mindestens eventuell mindestens ein Radikal trägt, wie unter a) definiert,
c) ein alicyclisches Radikal oder ein aromatisches Radikal mit eventuell mindestens einer Seitenkette, die ein Heteroatom umfasst oder eventuell mindestens ein Heteroatom in dem Ring umfasst,
d) ein Radikal, wie hier oben unter a), b) und c) definiert und ferner Halogenatome in halogenierter oder perhalogenierter Form tragen.

2. Verbindung nach Anspruch 1, **dadurch gekennzeichnet, dass** das organische Kation ein Oniumkation ist, das aus der Gruppe ausgewählt ist, die von den Kation R₃O⁺ (Oxonium), NR₄⁺ (Ammonium), RC(NHR₂)₂⁺ (Amidinium), C(NHR₂)₃⁺ (Guanidinium), C₅R₆N⁺ (Pyridinium), C₃R₅N₂⁺ (Imidazolium), C₃R₇N₂⁺ (Imidazolinium), C₂R₄N₃⁺ (Triazolium), SR₃⁺ (Sulfonium), PR₄⁺ (Phosphonium), IR₂⁺ (Iodonium), (C₆R₅)₃C⁺ (Carbonium) gebildet wird, den R-Radikalen, die unabhängig ein H darstellen oder ein Radikal, das aus der Gruppe ausgewählt ist, die gebildet wird von:
- den Alkyl-, Alkenyl-, Oxaalkyl-, Oxaalkenyl-, Azaalkyl-, Azaalkenyl-, Thiaalkyl-, Thiaalkenyl-, Silaalkyl-, Silaalkenyl-, Aryl-, Arylalkyl-, Alkylaryl-, Alkenylarylradikalen, den Dialkylaminoradikalen und den Dialkylazoradikalen,
- den cyclischen oder heterocyclischen Radikalen, die eventuell mindestens eine Seitenkette umfassen, die Heteroatome wie den Stickstoff, den Sauerstoff, den Schwefel umfassen,
- den cyclischen oder heterocyclischen Radikalen, die eventuell Heteroatome im aromatischen Kern umfassen,
- den Gruppen, die mehrere aromatische oder heterocyclische Kerne umfassen, kondensiert oder nicht, die eventuell mindestens ein Stickstoff-, Sauerstoff-, Schwefel- oder Phosphoratom enthalten,
wobei es sich versteht, dass mehrere R-Radikale gemeinsam aliphatische oder aromatische Ringe bilden können, die eventuell das Zentrum umschließen, das die kationische Ladung trägt.

3. Verbindung nach Anspruch 2, **dadurch gekennzeichnet, dass** das Oniumkation Bestandteil des Z-Radikals ist.

4. Verbindung nach Anspruch 2, **dadurch gekennzeichnet, dass** das Oniumkation Teil einer sich wiederholenden Einheit eines Polymers ist.

5. Verbindung nach Anspruch 2, **dadurch gekennzeichnet, dass** das Oniumkation eine heterocyclische kationische Gruppe mit aromatischem Charakter umfasst, die mindestens ein alkyliertes Stickstoffatom in dem Ring aufweist.

6. Verbindung nach Anspruch 5, **dadurch gekennzeichnet, dass** das Oniumkation eine Imidazoliumgruppe, eine Triazoliumgruppe, eine Pyridiniumgruppe, eine 4-Dimethylamin-pyridiniumgruppe umfasst, wobei die Gruppen eventuell einen Substituenten auf den Kohlenstoffatomen des Rings tragen.

7. Verbindung nach Anspruch 2, **dadurch gekennzeichnet, dass** das Oniumkation eine Gruppe ist, die eine Verbindung -N=N-, -N=N+, eine Sulfoniumgruppe, eine Iodoniumgruppe oder ein Aren-Ferrocen-Kation, substituiert oder nicht, eventuell in ein Polymergerüst inkorporiert, besitzt.

8. Verbindung nach Anspruch 7, **dadurch gekennzeichnet, dass** das Oniumkation ein Diaryliodoniumkation, ein Dialkylaryliodoniumkation, ein Triarylsulfoniumkation, ein Trialkylarylsulfoniumkation oder ein Phenacyldialkylsulfoniumkation, substituiert oder nicht, ist.

9. Verbindung nach Anspruch 2, **dadurch gekennzeichnet, dass** das Oniumkation Teil eines Polymers ist.

10. Verbindung nach Anspruch 2, **dadurch gekennzeichnet, dass** das Oniumkation eine 2,2'[Azobis(2-2'-imidazolinio-2-yl)propan]²⁺- oder 2,2'-Azobis(2-amidinio-propan)²⁺-Gruppe umfasst.

11. Verbindung nach Anspruch 1, **dadurch gekennzeichnet, dass** das Kation ein Metallkation ist, das aus der Gruppe ausgewählt ist, die von den Kationen alkalischer Metalle, den Kationen erdalkalischer Metalle, den Kationen von Übergangsmetallen und den Kationen dreiwertiger Metalle gebildet wird.

12. Verbindung nach Anspruch 1, **dadurch gekennzeichnet, dass** das Kation ein Metallocenium ist, das aus der Gruppe ausgewählt ist, die von den Kationen gebildet wird, die von dem Ferrocen, dem Titanocen, dem Zirconocen, den Indenoceniumkationen, den Aren-Metallocenium-Kationen, den Kationen der Übergangsmetalle, die von den Liganden vom Typ Phosphine komplexiert wurden und eventuell eine Chiralität besitzen, und den Organometallkationen, die eine oder mehrere Alkyl- oder Arylgruppen besitzen, die kovalent an einem Atom oder einer Atomgruppe fixiert sind, abgeleitet sind, wobei die Kationen eventuell Teil einer Polymerkette sind.

13. Verbindung nach einem der vorangehenden Ansprüche, **dadurch gekennzeichnet, dass** die R_{A}- und R_{B}-Substituenten unabhängig voneinander aus den Alkylradikalen und den Alkenylradikalen ausgewählt sind, die 1 bis 10 Kohlenstoffatome haben.

14. Verbindung nach einem der vorangehenden Ansprüche, **dadurch gekennzeichnet, dass** die R_{A}- und R_{B}-Substituenten unabhängig voneinander aus den halogenierten oder perhalogenierten Alkylradikalen oder Alkenylradikalen ausgewählt sind, die 1 bis 10 Kohlenstoffatome haben.

15. Verbindung nach einem der vorangehenden Ansprüche, **dadurch gekennzeichnet, dass** die R_{A}- und R_{B}-Substituenten unabhängig voneinander aus den Oxaalkyl- oder Oxaalkenylradikalen ausgewählt sind, die 1 bis 10 Kohlenstoffatome haben.

16. Material mit Ionenleitung, das eine in einem Lösungsmittel gelöste Ionenverbindung umfasst, **dadurch gekennzeichnet, dass** die Ionenverbindung eine Verbindung nach Anspruch 1 ist.

17. Elektrochemischer Generator, der eine negative Elektrode und eine positive Elektrode umfasst, die durch ein Elektrolyt getrennt sind, **dadurch gekennzeichnet, dass** das Elektrolyt ein Material nach Anspruch 1 ist.

18. Elektrisches Leitungsmaterial, **dadurch gekennzeichnet, dass** es eine Verbindung nach Anspruch 1 enthält.
